# EUROPEAN PATENT SPECIFICATION

(11) **EP 0 304 578 B1**
(45) Date of publication and mention of the grant of the patent: **24.10.2001**
(21) Application number: 88109946.9
(22) Date of filing: 22.06.1988
(51) Int. Cl.: C12N 15/00, A61K 39/29

(54) **Peptide comprising hepatitis B surface antigen**
Hepatitis-B-Oberflächenantigen enthaltendes Peptid
Peptide contenant l'antigène de surface de l'hépatite B

(30) Priority: 22.06.1987 EP 87108915; 22.06.1987 EP 87108914
(43) Date of publication of application: 01.03.1989
(60) Divisional application: 00121167.1
(73) Proprietor: MEDEVA HOLDINGS B.V., 1078 ED Amsterdam (NL)
(72) Inventor: Thoma, Hans A. Dr., D-8000 München 40 (DE)
(74) Representative: Woods, Geoffrey Corlett

(56) References cited:
- EP-A- 0 156 712
- EP-A- 0 180 012
- EP-A- 0 198 474
- EP-A- 0 244 924
- EP-A- 0 248 410
- EP-A- 0 250 253
- WO-A-86/05189
- JOURNAL OF IMMUNOLOGY, vol. 138, no. 12, 15th June 1987, pages 4457-4465, The American Association of Immunologists, US; D.R. MILICH et al.: "A single 10-residue pre-S(1) peptide can prime T cell help for antibody production to multiple epitopes within the pre-S(1), pre-S(2), and S regions of HBsAg1"
- JOURNAL OF GENETIC VIROLOGY, vol. 67, 1986, pages 2305-2314, SGM, GB; H. OKAMOTO et al.: "Nucleotide sequence of a cloned hepatitis B virus genome, subtype AYR: comparison with genomes of the other three subtypes"
- JOURNAL OF IMMUNOLOGY, vol. 137, no. 8, 15th October 1986, pages 2703-2710, The American Association of Immunologists, US; D.R. MILICH et al.: "Two distinct but overlapping antibody binding sites in the pre-S(2) region of HBsAg localized within 11 continuous residues"
- SCIENCE, vol. 228, 7th June 1985, pages 1195-1199; D.R. MILICH et al.: "Enhanced immunogenicity of the pre-S region of hepatitis B surface antigen"
- PROCEEDINGS OF THE NATIONAL ACADEMY OF SCIENCES, vol. 81, no. 24, December 1984, pages 7708-7712, Washington, US; M.-L. MICHEL et al.: "Synthesis in animal cells of hepatitis B surface antigen particles carrying a receptor for polymerized human serum albumin"

## Description

The invention relates to Hepatitis B surface antigen ("HBs antigen" or "HBsAg") polypeptides prepared by recombinant DNA processes, DNA sequences coding for these polypeptides end cell lines for the expression of the same.

### BACKGROUND OF THE INVENTION

### EXPRESSION IN HOST CELLS

Advances in vaccine production techniques have made it possible to synthesize polypeptides corresponding to the HBs antigen in bacteria, yeast and mammalian cells. Transcription of eukaryotic genes in bacteria and yeast, however, adversely affects the efficaciousness of these polypeptides as antigens due to several drawbacks concerning the glycosylation and secretion of the polypeptides and composition of the particle formed therefrom.

For example, in the case of the Hepatitis B virus, the polypeptide antigens produced in vivo are heavily glycosylated (Gerlich, 1984: J. Virol.: 52 (2), 396). In prokaryotes, glycosylation is not an essential process so that polypeptides produced by genetically engineered bacteria are either not glycosylated or are incompletely glycosylated. In either case, polypeptides corresponding to HBsAg, when expressed in bacteria, do not raise antibodies which will see HBsAg sufficiently well for an effective vaccine. Although yeast as a eukaryotic host is capable of more complete glycosylation, polypeptides corresponding to HBsAg expressed in yeast share the same deficiency as in the case of bacterial expression (Murray et al., 1979: Nature, 282, 575; Valenzuela et al., 1982: Nature, 298, 347; Miyanohara et al., 1983: PNAS, 80, 1).

As a further example, in bacteria the eukaryotic structural gene of the HBsAg is in most cases not efficiently transcribed. Furthermore the structure and function of the eukaryotic HBsAg gene product may be dependent on the additional post-translational processes of the linkage of disulfide bonds which can not be accomplished by the bacterial host.

Still further, the expressed polypeptide is rarely secreted from the bacterial host cells. They must be lysed to harvest the expressed polypeptide. During the purification process bacterial wall components may contaminate the polypeptide and cause serious allergic reactions or lead to anaphylactic shock in patients.

Finally, eukaryotic promoters usually do not work in bacteria and must be substituted by a bacterial promoter which can result in modification of the polypeptide expressed. (Offensperger et al., 1985: PNAS, 82, 7540; Valenzuela et al., 1980: ICN-UCLA Symp, Mol. Cell. Biol., 18 57).

### FORMATION AND SECRETION OF PARTICLES

The natural forms of Hepatitis B virus ("HBV") and HBV protein occur in three distinct morphologies:
- the HBV-virion (Dane particle), which is thought to be the infectious material,
- the filaments, and
- the 20 or 22 nm particles (hereinafter "20 nm particle") which consist only of a protein envelope.

The most interesting form for an efficient vaccine is the 20 nm particle because 1) the coding sequences are entirely known, 2) it is completely uninfectious, and 3) it causes some useful immunogenicity in a human organism.

The three known components of HBV particles differ in their relative amounts of the protein composition. There are three monomers called the major protein with 226 amino acids, the middle protein with 281 amino acids, and the large protein with 389 or 400 amino acids, depending on the subtype ayw and adw, respectively. The large protein is encoded by the complete sequence of the pre-S₁-, pre-S₂- and S-regions, whereas the middle protein is derived from only the pre-S₂- and S-regions, and finally the major protein from only the S-region (Tiollais et al., 1985: Nature, 317, 489; Dubois et al., 1980: PNAS, 77, 4549; McAlzer et al., 1984: Nature, 307, 178).

The infectious virion of HBV (Dane particle) contains 40-80 times more of the high molecular monomers - the pre-S₁ and pre-S₂ peptides - compared to the 20 nm particle. It is now known that these pre-S polypeptides may be associated with some biological and clinical implications. The polyalbumin receptor on the pre-S polypeptides can bind polymerized albumins from humans and chimpanzees which are susceptible to HBV (Thung et al., 1983: Liver, 3, 290; Machida et al., 1984: Gastroenterology, 86, 910). This narrow host range and the known receptor for poly human serum albumin on human hepatocytes explain the hepatotropism of HBV: Dane particles are able to contact hepatocytes via poly human serum albumin taken up by hepatocytes from circulation. Based on this evidence the pre-S peptides should be helpful for an efficient vaccine against HBV because its antibody could be expected to block the significant site on Dane particles that are required for entering hepatocytes (Tiollais et al., 1985: Nature, 317, 489; Millich et al., 1985: Science, 228, 1195).

Literature data would also suggest a better protection against the infectious Dane-particle where the pre-S₁ epitope is present in much higher ratio than on the envelope particles.

The vaccine obtained from natural sources (e.g., donor blood), which causes a limited immunogenic protection, contains (almost) none of the pre-S proteins; this is due to two different reasons. First, the purification process is focused on the noninfectious 20 nm particles. These contain at most 1% pre-S₁ peptide compared to 15-20% in the Dane particle (Gerlich, 1984: J. Vir., 52 (2), 396; Tiollais et al., 1985: Nature, 317, 489; Gerlich, 1982: virology, 123, 436). Second, the 20 nm particles are isolated from sera of anti-HBe positive carriers (Hevac B, HepaVac B) or are digested by proteases during the purification process. This proteolytic digestion has been shown to cut the pre-S-polypeptides leaving only the S monomers. As a result these vaccines contain none or very little pre-S polypeptides.

Therefore there is a demand for a vaccine in the form of HBs antigen particles which possess a high immunogenicity due to the composition of the particle, which undergo glycosylation in the cell and which are secreted continuously from the particle-producing cell.

### REFERENCES AND PATENTS

EP-A-72 318 describes the expression of HBsAg in yeast cells, which have been transformed by a vector comprising a yeast replicon, a yeast promoter and a DNA sequence coding for the S peptide.

Laub et al., J. Virol., Vol. 48, No. 1, pp. 271-280, 1983, disclose the construction of a vector starting from simian virus 40 into which the HBsAg including the 163 codon precursor sequence was incorporated. Laub et al. report that CV-1 cells transformed with said vector yield a better expression when the vector contains only the coding sequence for the S protein as compared to the above vector which comprises additionally also the 163 codon precursor sequence.

Also Takeda Chemical Ind., Japanese Patent Application No. J5-8194-897-A describes the expression of the entire pre-S and S peptides. Reference is also made to the expression of the adw subtype.

Feitilson et al., Virology, Vol. 130, pp. 75-90, 1983, have described the partial expression of polypeptides within the pre-S coding sequence, including species with 24000, 28000, 32000, 43000 and 50000 dalton.

Further, DE-OS 34 39 400 describes the expression of an immunogenic polypeptide sequence of Hepatitis B virus.

Said sequence represents a partial sequence of the pre-S₁ polypeptide, comprises 108 or 119 codons and starts with the first starting codon of HBsAg, and terminates 281 codons in front of the stop codon.

EP-A-154 902 discloses a Hepatitis B vaccine which contains a peptide with an amino acid chain of at least six consecutive amino acids within the pre-S chain coding region of the envelope of Hepatitis B virus. This vaccine is free of an amino acid sequence corresponding to the naturally occurring envelope proteins of Hepatitis B virus.

Also Kent et al. have described in Pept. Chem., Vol 22, pp. 16770, 1984, that a chemically synthesized peptide comprising the N-terminal 26 amino acids of the pre-S₂ region can serve as an antigen and may therefore be suitable as a synthetic vaccine.

### OBJECTS OF THE INVENTION

None of the above discussed references consider the possibility that, by altering the composition of the monomers making up the 20 nm particles and approaching thereby the natural composition of the Dane particle, the antigenicity of the particle can be improved.

As discussed mentioned above, the immunogenicity of the peptide monomers of the virus envelope protein is very poor compared to assembled protein particles. The object of this invention is the development of protein particles which contain an amount of the pre-S polypeptide epitopes comparable to the natural composition of the surface structure of the infectious Dane particle.

It is a further object to utilize additional pre-S peptides containing important protective epitopes in the development of a better immune response, a longer protection and lower non-responder rate as compared to all the other products either already marketed or under development.

It is a further object to express HBsAg in mammalian cells. This requires overcoming known difficulties where expression of the desired peptide in a mammalian cell can result in:
- different regulatory mechanisms for the three translational/(transcriptional) products
- promoter-promoter inhibition
- different strength of the start codons
- not all peptides expressed.

### SUMMARY OF THE INVENTION

The term "HBV S peptide" as used herein refers to the peptide encoded by the entire S region of the HBV genome. The term "HBV pre-S₂ peptide" as used herein refers to the peptide encoded by the entire pre-S2 and S regions of the HBV genome. The term "HBV pre-S₁ peptide" as used herein refers to the polypeptide encoded by the entire pre-S₁, pre-S₂ and S regions of the HBV genome. The term "epitope" as used herein refers to a sequence of at least six consecutive amino acids encoded by the designated genome region (e.g., a "HBV pre-S₂ epitope" refers to a sequence of at least six amino acids encoded by the pre-S₂ region of the HBV genome). As used herein "antigenicity" means the ability to provoke an immune response (e.g., acting as a vaccine or an antigen), the ability to cause the production of antibodies (e.g. acting as an antigen) and/or the ability to interact with a cell surface receptor so as to enhance an immune response or production of antibodies (e.g., reacting with a T-cell surface receptor to enhance immune response).

The term "HBV" means any subtype of the virus, particularly adw, ayw, adr and ayr, described in the literature (P. Valenzuela, Nature Vol. 280, p. 815 (1979), Gerlich, EP-A-85 111 361, Neurath, EP-A-85 102 250). Examples of peptide sequences thereof, from which the epitopes of this invention can be derived, are shown in Figures XVI to XX.

The present invention provides a recombinant DNA molecule comprising an expression control sequence operatively linked to first and second DNA sequences linked in frame, said DNA sequences each encoding a discrete region of a single peptide expressed by said recombinant DNA molecule, and said first DNA sequence being closer to said expression control sequence than said second DNA sequence, characterised in that:
(a) said first DNA sequence comprises the nucleotide sequence of part of the HBV pre-S₁ coding region wherein a 5'- terminal region of said pre-S₁ coding region containing the pre-S₁ region ATG start codon has been replaced by a 5'- terminal coding region of the HBV S region containing the S region ATG start codon, and said first DNA sequence encodes an HBV pre-S₁ epitope; and
(b) said second DNA sequence comprises nucleotide sequence of the HBV S coding region wherein the ATG start codon is absent and encodes a peptide which upon secretion will direct formation of particles that will provoke an immune response against the pre-S₁ epitope.

Host cells transfected with the recombinant DNA molecules of the present invention are also disclosed. As used herein, "transfected" or "transfection" refers to the addition of exogenous DNA to a host cell whether by transfection, transformation or other means. Host cells include any unicellular organism capable of transcribing and translating recombinant DNA molecules including without limitation mammalian cells, bacteria and yeast. Host cells of the present invention may also be cotransfected with a second recombinant DNA molecule encoding a peptide including the amino acid sequence of the HBV S peptide.

The invention includes peptides encoded by the recombinant DNA molecules of the invention.

Immunogenic particles are also disclosed which comprise a plurality of first peptide monomers encoded by a recombinant DNA molecule of the invention. Immunogenic particles are also disclosed which further comprise a plurality of second peptide monomers and wherein the first and second peptide monomers are bound together by interactive forces between the monomers. Each of said second peptide monomers comprises the amino acid sequence of the HBV S peptide.

Immunogenic particles are also disclosed which contain substantially more than one percent, preferably more than five percent, of the pre-S₁ epitope. As used herein, a particle "contains one percent" of a designated epitope if peptide monomers having the designated epitope constitute one percent of all protein in the particle. Immunogenic particles which contain substantially more than ten percent, preferably more than fifteen percent, of the pre-S₂ epitope are also disclosed.

Pharmaceutical preparations and preparations useful for production of antibodies comprising the above-described immunogenic particles in sufficient concentration to elicit an immune response upon administration of said preparation and a suitable carrier are also disclosed. Suitable carriers are known to those skilled in the art and may include simple buffer solutions.

Other preparations useful for production of antibodies are disclosed comprising the above-described immunogenic particles in sufficient concentration to elicit an immune response upon administration of said preparation and a suitable carrier. Suitable carriers are known to those skilled in the art and may include simple buffer solutions.

A process for producing a transfected host cell is disclosed which comprises providing a host cell which has been made competent for uptake of DNA, exposing the host cell to a first preparation of DNA comprising a recombinant DNA molecule of the invention, allowing under suitable conditions the host cell to take up DNA from the first preparation of DNA, and selecting for a host cell which has taken up the recombinant DNA molecule. The process may further comprise exposing the host cell to a second preparation of DNA comprising a DNA molecule encoding a peptide including the amino acid sequence of the HBV S peptide and allowing under suitable conditions the host cells to take up DNA from the second preparation of DNA. The exposure and uptake of the second preparation of DNA. The exposure and uptake of the second preparation of DNA can be done before or after exposure to and uptake of the first DNA preparation. Alternatively, the first DNA preparation can also include a DNA molecule encoding for a peptide including the amino acid sequence of the HBV S peptide.

A method for producing a peptide is also disclosed which comprises culturing a host cell transfected with a recombinant DNA molecule of the invention under conditions allowing expression and secretion of protein by the host cell, and collecting the peptide produced as a result of expression from the recombinant DNA molecule.

A method of producing immunogenic particles is disclosed comprising preparing an above-described recombinant DNA molecule of the invention, transfecting a host cell with the recombinant DNA molecule, culturing the host cell under conditions allowing expression and secretion of protein by the host cell, and allowing under suitable conditions the aggregation of peptide monomers produced as a result of expression of exogenous DNA sequences within the host cell. A method of producing immunogenic particles is also disclosed which further comprises transfecting (cotransfecting) the host cell with a DNA molecule encoding a peptide including the amino acid sequence of the HBV S peptide. The cotransfection can occur before, after or simultaneous with the transfection of the above-described recombinant DNA molecule. The presence of peptides encoded by the cotransfected DNA molecule is necessary to obtain more than trace amounts of particles secreted from the host cell.

Methods of manufacturing a pharmaceutical preparation and a preparation useful for production of antibodies are disclosed comprising preparing an above-described recombinant DNA molecule of the invention, transfecting a host cell with the recombinant DNA molecule, culturing the host cell under conditions allowing expression and secretion of protein by the host cell, allowing under suitable conditions the aggregation of peptides produced as a result of expression of DNA sequences within the host cell to form immunogenic particles, and combining the immunogenic particles with a suitable carrier such that the immunogenic particles are present in sufficient concentration to cause production of antibodies upon administration of a preparation to an individual. Host cells used in these methods can also be cotransfected as previously described.

### BRIEF DESCRIPTION OF THE FIGURES

Figures I, III, IV, VI and VIII show gene constructs according to the present invention.

Figures II, V, VII and X show gene constructs which are outside the scope of the present invention, and which encode pre-S2-containing peptides.

The shaded areas in Figures I to VIII and X indicate regions that are not present in the constructs.

Figure IX shows a BglII-BglII sequence coding for pre-S₁, pre-S₂ and S.

Figures XI and XII show the results obtained by caesium chloride sedimentation of immunogenic particles according to example 10.

Figure XIII shows a prior art construct shown in Table XI.

Figure XIV shows the characterisation of particles with pre-S₁ and pre-S₂ epitopes.

Figure XV shows the oligo sequence of Figure X-2.

Figures XVI to XX show examples of peptides sequences from which the epitopes used in this invention can be derived.

### DESCRIPTION OF THE PREFERRED EMBODIMENTS

Preferred DNA constructs of the present invention are characterized by the presence of a selection marker selected from the group consisting of dhfr (dihydrofolate reductase), MT-neo (a neomycin resistance sequence coupled to a methallothionein promoter) and MT-ecogpt (a resistance sequence coupled to a methallothionein promoter). The expression rate may be further enhanced by adding to the constructs a dhfr gene as an amplification gene.

HBV nucleotide sequences used in certain constructs of the present invention can be formed or isolated by any means including isolation and ligation of restriction fragments and synthetic oligonucleotides. Constructs specifically described herein were formed by the ligation of synthetic oligonucleotides to a 5' XbaI-BglII 3' fragment from the S region of the HBV genome shown in Figure IX (hereinafter the "XbaI-BglII fragment") which is derived from a BglII-BglII HBV fragment including the entire pre-S₁-pre-S₂-S regions (the "BglII-BglII Fragment"). The pre-S₁-pre-S₂-S region of the HBV genome is shown in Figure IX, Oligonucleotides used in making such constructs are summarized in Table I.

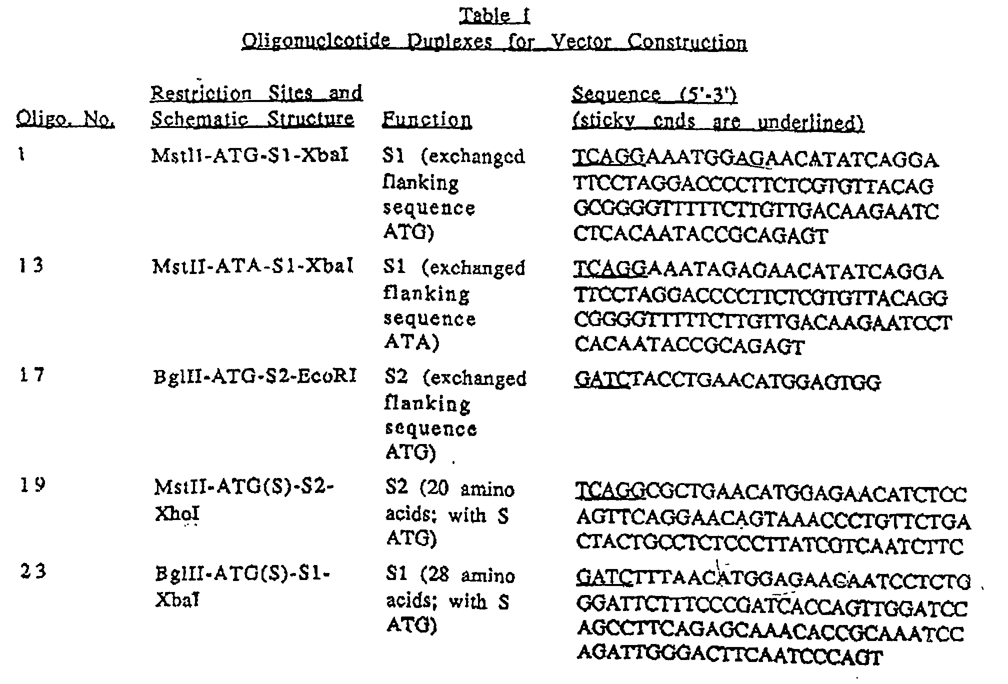

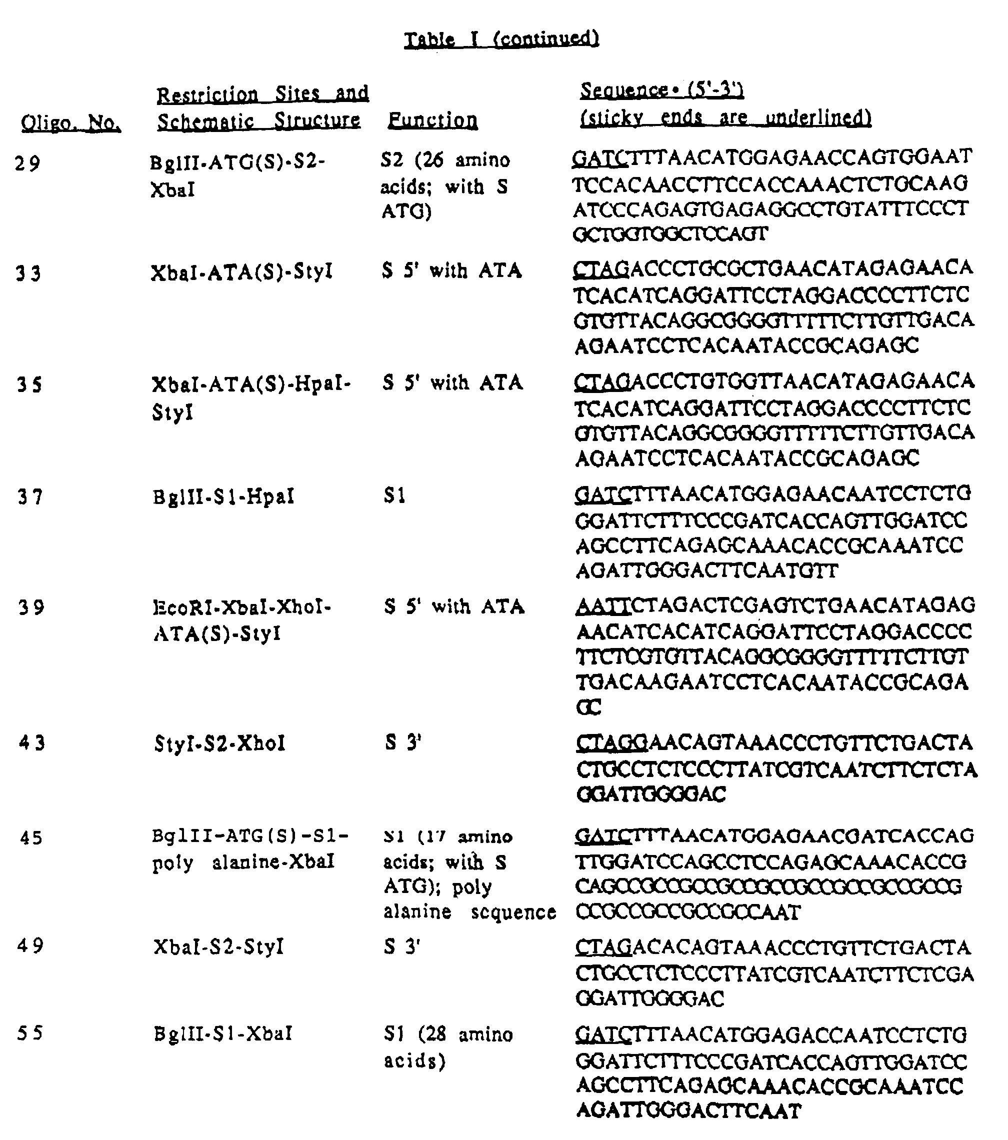

The oligonucleotides in Table I were combined with the XbaI-BglII fragment to produce constructs with desired features. In certain constructs adapter oligonucleotide sequences (Table II) were used to create proper matching sticky ends on the oligonucleotides and other construct components.

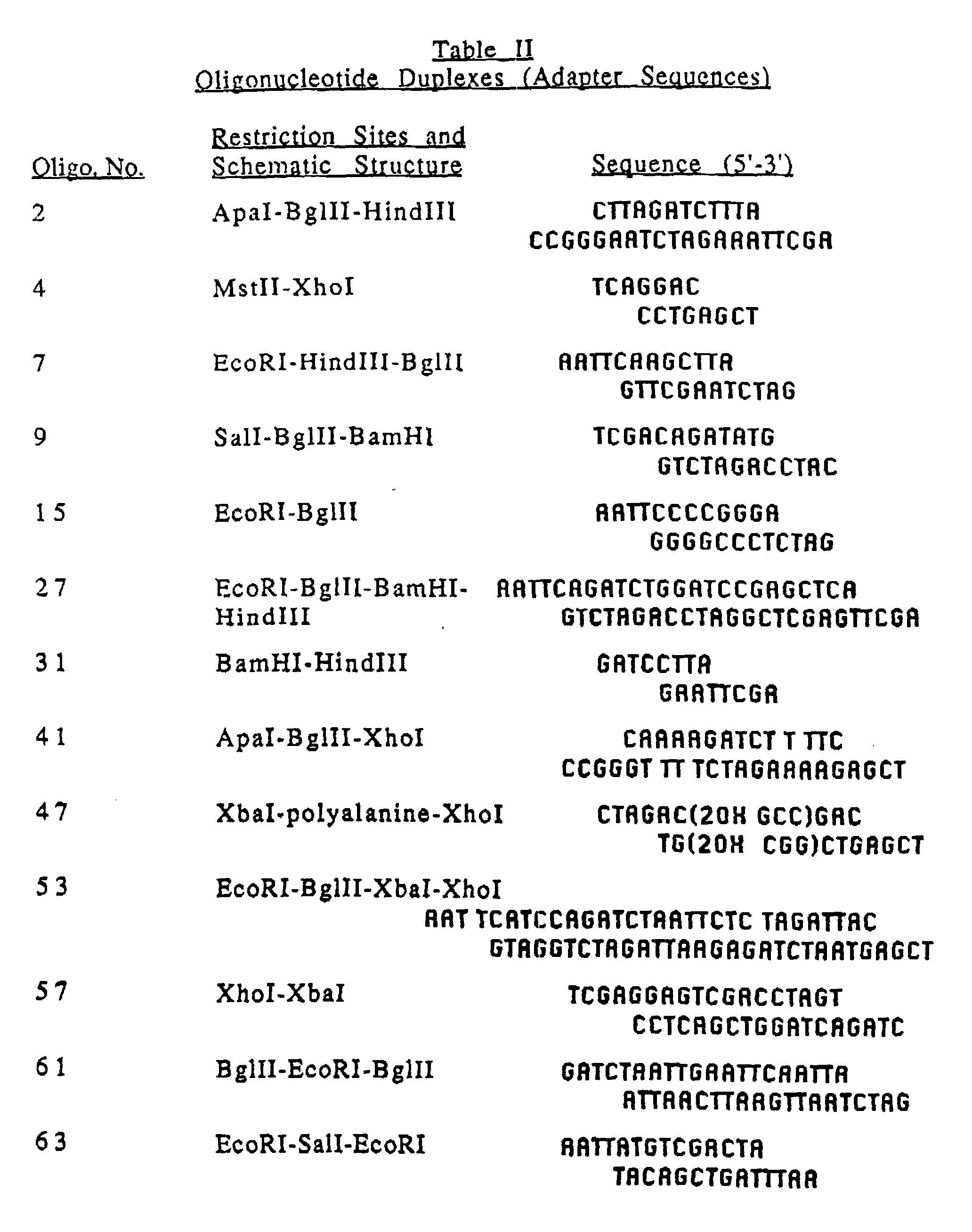

Other adapter sequences may be used to combine desired oligonucleotides from Table I with the XpaI-BglII fragment, other restriction fragments, oligonucleotides and other construct components. The necessary sequences of such other adapter sequences will be readily apparent to those skilled in the art from consideration of tables of restriction sites [e.g., that found at pages 121-128 of Methods in Enzymology, volume 152, "Guide to Molecular Cloning Techniques," ed, Berger and Kimmel (Academic Press 1987) which is incorporated herein in its entirety by reference] and the sequences of the various nucleotides to be combined. Adapter sequences can also be used to introduce additional restriction sites into constructs of the present invention. It should be noted that adapter sequences must be selected or designed so that the proper reading frame is maintained throughout the HBV sequence.

Preferred gene constructs which were used to transfect host cells were prepared by recombinant DNA techniques in accordance with the present invention. Preferred embodiments of constructs with an enhanced expression rate are shown in Figures I-VIII and are schematically represented by the following:
pU2-structural gene
pU2-structural gene-dhfr
pU2-structural gene-dhfr-MT-neo
pU2-structural gene-dhfr-MT-egpt
pMT-structural gene-dhfr
pMT-structural gene-dhfr-MT-neo
pMT-structural gene-dhfr-MT-egpt
pH2K-structural gene-dhfr
pH2K-structural gene-MT-neo
pH2K-structural gene-MT-egpt
pH2K-structural gene-dhfr-MT-neo
pH2K-structural gene-dhfr-MT-egpt

Each of the constructs shown in Figures I-VIII contain, in addition to a HBV sequence, a neomycin selection marker with the MT promoter, an ampicillin selection marker, a dhfr selection/amplification gene and a promoter for the HBV sequence. The promoter for the HBV sequence is preferably the U2 promoter, the MT promoter or the H2K promoter. Isolation of fragments containing the various promoters, the selection markers and amplification gene is described below. The HBV sequences in the constructs of Figures I-VIII are schematically represented by a rectangular bar in each figure which indicates the oligonucleotides and/or adapter sequences from Tables I and II which were combined with the XbaI-BglII fragment. Shaded areas within the bar indicate generally regions of the entire pre-S₁-pre-S₂-S region which are. not found in the specific construct. Oligonucleotides from Table I which can be used to construct each type of HBV sequence are indicated in the figures.

Figure X depicts two additional constructs for expression of peptides including sequence from the pre-S2 region under the control of the MT promoter.

Constructs have also been made which include the entire BglII-BglII fragment from the HBV genome under the control of the U2 promoter. These constructs have produced peptides which include a deletion in the S region as indicated by Western blot analysis.

The above-cited promoters are specially preferable when their use is coupled with a modulation method using the dhfr gene and methotrexate to enhance the expression. This is achieved when in addition to the selection marker the dhfr minigene is also introduced into the plasmid sequence. It is essential that the dhfr gene is located on the same plasmid together with the structural gene to be expressed. An enhancement of the expression rate of the structural gene can then be obtained by adding methotrexate in the micromolar concentration range. Thereby a manyfold enhancement of the expression rate is achieved.

Suitable cells are e.g. VERO cells (monkey kidney cell line), 3T3-cells (murine fibroblast line), C127-cells (murine fibroblast line), L-cells and CHO - cells (Chinese hamster cells, which are either positive or negative in dehydrofolate reductase).

As a stop signal it is preferred to use a stop signal from a eukaryotic cell. Preferably the stop signal of the caseine DNA-sequence is used. As used throughout the following examples, "HBV protein" refers generically to any protein produced in accordance with the present invention which corresponds to HBsAg sequences.

### EXAMPLE 1

### Particle Purification Procedures

### 1. Fractionated precipitation with polyethylene glycol (PEG)

The supernatant of HBV protein producing cultures was collected and split into portions of 2,400 ml. To each portion 144 g of PEG 6000 (Serva) were added and dissolved by stirring at room temperature for 20 minutes and was stirred for another 6 hours at 4°C. The precipitate was separated by centrifugation in 500 ml bottles in a GS 3 rotor at 9,000 rpm (15,000 x g) for 30 minutes at 10 C. The supernatant was collected and 144 g of PEG 6000 were added and dissolved as described above. The solution was stirred at 4 C for 3 hours. The precipitate from this solution was harvested as described above except that centrifugation was continued for 60 minutes.

### 2. Gel Chromatography

The material obtained after PEG precipitation was redissolved in 20 ml PBS and submitted to gel chromatography on A-5m (BioRad). Column dimensions were 25 x 1000 mm and 480 ml bed volume. In a typical fractionation run 1,000 ug of PEG precipitated HBV protein in 10 to 15 ml was loaded and eluted with PBS at a speed of 6 drops/min (18 ml/h) 3 ml fractions were collected. HBV protein eluted with the first peak. Collected fractions were submitted to a CsCl gradient.

### 3. Sedimentation in CsCl Gradient

About 30 fractions covering the first peak in column chromatography on A-5m and containing prepurified HBV protein were collected to approximately 100 ml. This solution was adjusted to a density of 1.30 g/cc with CsCl and subsequently transferred to a nitrocellulose tube fitting into a SW 27/28 rotor (Beckman). A gradient was set by underlaying 4 ml of a CsCl solution of 1.35 g/cc and by overlaying 4 ml of 1.25 g/cc followed by 4 ml of 1.20 g/cc density. This gradient had been run at 28,000 rpm for 50 hours at 10 C. Thereafter the gradient was fractionated and purified HBV protein floating in the 1.20 g/cc density layer was collected. The solution was desalted by three cycles of dialysis in bags against water.

### Example 2

### Quantitative Determination of HBV protein

### 1. with Radioimmunoassay

In the AUSRIA II-125 "sandwich" radioimmunoassay (commercially available from Abbot), beads coated with guinea pig antibody to Hepatitis B Surface Antigen (Anti-HBs) were incubated with serum or plasma or purified protein and appropriate controls. Any HBsAg present was bound to the solid phase antibody. After aspiration of the unbound material and washing of the bead, human 125T-Anti-HBs was allowed to react with the antibody-antigen complex on the bead. The beads were then washed to remove unbound ¹²⁵I-Anti-HBs.
)-Anti-HBs HBsAg
)-Anti-HBs . HBsAg 125I-Anti-HBs
)-Anti-HBs . HBsAg . 125-Anti-HBs

The radioactivity remaining on the beads was counted in a gamma scintillation counter.

### 2. with ELISA

In the Enzygnost HBsAg micro "sandwich" assay (commercially available from Behring), wells were coated with anti-HBs. Serum plasma or purified protein and appropriate controls were added to the wells and incubated. After washing, peroxidase-labelled antibodies to HBsAg were reacted with the remaining antigenic determinants. The unbound enzyme-linked antibodies are removed by washing and the enzyme activity on the solid phase is determined. The enzymatically catalyzed reaction of hydrogen peroxide and chromogen was stopped by adding diluted sulfuric acid. The colour intensity was proportional to the HBsAg concentration of the sample and was obtained by photometric comparison of the colour intensity of the unknown samples with the colour intensities of the accompanying negative and positive control sera.

### Example 3

### Preparation of a construct of the present invention containing the methallothionein promoter.

### 1) Isolation of the MT promoter

The plasmid pBPV-342-12 (commercially available from ATCC) was digested with the endonucleases BglII and BamHI. Three DNA molecules were generated. The fragment of interest contains the methallothionein promoter and a pBR322 sequence comprising 4.5 kb and is easily detectable from the other fragments (2.0 kb and 7.6 kb).

The reaction was performed in a total volume of 200 ul of reaction buffer at a final concentration of 0.5 ug/ul DNA including 100 units of each restriction enzyme. The completion of the digestion was checked after incubation at 37°C for three hours by agarose gel electrophoresis at a 0.8% agarose gel. The reaction was stopped by adding 4 ul 0.5 M EDTA.

The 4.5 kb fragment was separated from the other fragments by preparative 1.2% agarose gel electrophoresis. The DNA was eluted from the agarose gel on DE-81 whatman filter paper from which the DNA was removed in a high salt buffer. The DNA was purified by a phenol/chloroform extraction and two ethanol precipitations.

### 2) Ligation of the 2.3 kb HBV BglII-BglII fragment

A 2.3 kb BglII-BglII fragment containing the HBV pre-S₁,pre-S₂ and S coding regions was isolated from HBV-containing DNA. The 2-3kb fragment was ligated together with the 4.5 kb fragment (obtained as described in 1) containing the methallothionein promoter.

2 µl of the 2.3 kb fragment were mixed with 3 µl of the 4.5 kb fragment and ligated together in a total volume of 10 ul ligation buffer, containing 2 units T₄-DNA ligase and 2mM ATP at 14°C overnight.

The ligation mixture was added to 150 ul competent bacterial Cell suspension for DNA up-take. After the DNA up-date the bacterial cells were spread on LB agar plate containing 50 ug/ml ampicillin at volumes of 50 to 300 ul cell suspension per plate. The agar plates were incubated at 37°C overnight. Single isolated bacterial colonies were screened for the presence of a plasmid containing the desired fragments.

### 3) Screening for desired plasmid containing bacterial colonies.

Single colonies were picked with a toothpick and transferred to a LB-ampicillin media containing tube (5 ml). The tubes were incubated overnight at 37°C by shaking rapidly. A mini-plasmid preparation of each grown bacterial suspension was made. The different resulting DNAs were proved by digestion with the restriction endonuclease EcoRI. Two molecules were expected, a 2.2 kb fragment and a 4.6 kb fragment. The digestion was analysed by agarose gel electrophoresis. Plasmid DNA was isolated from the bacterial cells.

### 4) Conversion of a part of the HBV-gene sequence.

The plasmid resulting from (3) above was digested with the endonucleases BglII and XbaI. Two molecules were expected, one 550 bp fragment and one 6.250 kb fragment which was isolated after agarose gel electrophoresis.

The 6.250 kb fragment was ligated together with oligonucleotide No.55 from Table I. The ligation mixture was added to 150 ul competent bacterial cell suspension for DNA up-take. single isolated bacterial colonies were screened for the presence of the desired plasmid. The new plasmid was proved by a digestion with the endonucleases EcoRI and BglII. Two molecules were expected, one 1.9 kb and one 4.450 kb.

### 5) Insertion of a neomycin selection marker.

The plasmid resulting from (4) above was linearized by digestion with the restriction enzyme EcoRI. The reaction was performed in a total volume of 50 ul and a final concentration of 1 ug/ul plasmid DNA. 50 units of ECORI were added and the digestion was proved after incubation at 37°C for three hours by agarose gel electrophoresis. The reaction was stopped by adding 1 ul of 0.5 M EDTA and the DNA was precipitated with a final concentration of 0.3 M sodium acetate and 3-4 volumes of ethanol at -80°C for 30 minutes. The precipitated DNA was dissolved in 50 ul distilled water.

2 ul of the linearized plasmid were mixed with 3 ul of the DNA fragment containing the methallothionein promoter and the neomycin selection gene [isolated from the plasmid pMT-neo-E (available from ATCC ) by digestion with the endonuclease EcoRI as a 4kb fragment], and ligated together. Single bacterial colonies were screened for the presence of the desired plasmid.

### 6) Additional of the dhfr Amplification Gene dhfr

The plasmid pdhfr3.2 (available from ATCC) was digested with the restriction endonuclease HindIII. Two molecules were generated, one of 3,000 bp containing the dhfr gene sequence and one of 3,400 bp. The 3,000 bp fragment was isolated and ligated into the plasmid resulting from (5) above which was previously opened by digestion with HindIII. The resulting plasmid is represented by Fig. I-2.

### 1) Isolation of a fragment containing the U2 promoter sequence.

The plasmid pUC-8-42 (available from Exogene ) was digested with the restriction endonucleases EcoRI and Apal. Two DNA molecules were generated. The fragment of interest contains the U2-promoter comprising 340 bp and is easily detectable from the other fragment (3160 bp). The digestion was performed in a total volume of 200 ul of reaction buffer at a final concentration of 0.5 ug/ul DNA including 100 Units of each restriction enzyme. The completion of the digest was checked after incubation at 37°C for three hours by agarose gel electrophoresis in a 0.7% agarose gel. The reaction was stopped by adding 4 ul 0.5 M EDTA. The 340 bp fragment was separated from the plasmid DNA by preparative 1.2% agarose gel electrophoresis. The DNA was eluted from the agarose gel on DE-81 Whatman filter paper from which the DNA was removed in a high salt buffer. The DNA was purified by a phenol/chloroform extraction and two ethanol precipitations.

### 2) Insertion of the fragment containing the promoter sequence into a polylinker plasmid.

The plasmid pSP165 (commercially available from Promega Biotec) containing a polylinker sequence (containing the following restriction sites: EcoRI, SacI, Smal, AvaI, BamHI, BglII, Sail, PstI, HindIII) was linearized with the restriction enzyme EcoRI. The reaction was performed in a total volume of 50 ul and a final concentration of lug/ul plasmid DNA. 50 Units of EcoRI were added an the digestion was proved after incubation at 37°C for three hours by agarose gel electrophores. The reaction was stopped by adding 1 ul of 0.5 M EDTA and the DNA was precipitated with a final concentration of 0.3 M sodium acetate and 3-4 volumes of ethanol at -80°C for 30 minutes. The precipitated DNA was dissolved in 50 ul distilled water.

2 ul of plasmid DNA were mixed with 10 ul of the fragment DNA containing the u2 promoter sequence, and ligated together in a total volume of 25 ul of ligation buffer containing 2 units T4-DNA ligase and 2 mM ATP at 14°C overnight. Thereafter the DNA was purified by phenol/ chloroform extractions followed by two ethanol precipitations and dissolved in 10 ul distilled water. The resulting sticky ends of EcoRI and ApaI had to be converted into blunt ends and ligated. The blunt ends were converted by a removing reaction with the Mung bean nuclease as follows: to 25 ul DNA (1 ug/ul concentration) reaction buffer, 20 units of enzyme and a final concentration of 1% glycerol to the reaction volume of 35 ul were added. After an incubation for 30 minutes at 30 C the DNA was purified by phenol/chloroform extractions followed by two ethanol precipitations. The DNA was dissolved again in 5 ul distilled water. The resulting blunt ends were ligated together in 15 ul reaction volume containing 10 x more T4 ligase then used above and 2 mM ATP at 14°C overnight.

The ligation mixture was added to 150 ul competent bacterial cell suspension for DNA up-take. After the DNA up-take the bacterial cells were spread on LB agar plates containing 50 ug/ml ampicillin at volumes of 50 to 300 ul cell suspension per plate. The agar plates were incubated at 37°C overnight. Single isolated bacterial colonies were screened for the presence of a plasmid containing the desired U2-promoter fragment.

### 3. Screening for desired plasmid containing bacterial colonies

Single colonies were picked with a toothpick and transferred to a LB-ampicillin containing tube (5 ml). The tubes were incubated overnight at 37°C by shaking rapidly. A mini plasmid preparation of each grown bacterial suspension was made. The different resulting plasmid was proved by digestion with both restriction endonucleases EcoRI and HindIII. Two molecules were found, a 400 bp fragment containing the U2 promoter sequence and the plasmid of 2,700 bp. The digestion was analysed by agarose gel electrophoresis. The resulting plasmid was isolated from the bacterial cells.

### Insertion of the neomycin selection marker.

The plasmid pBPV-342-12 (commercially available from ATCC) was digested with the endonucleases EcoRI and BamHI. Two molecules were isolated, one containing the MT promoter together with the neomycin selection gene of 4,000 bp and the plasmid of 10,000 bp.

The plasmid resulting from (3) above was linearized with EcoRI and ligated together with the 4,000 bp fragment containing the MT-promoter together with the neomycin selection gene. The resulting sticky ends were also converted into blunt ends and ligated together as described above.

After bacterial transformation, colony selection and mini plasmid preparation, the resulting plasmids were analysed by a digestion with the restriction enzymes EcoRI and HindIII. Two DNA molecules were isolated, a 400 bp fragment and a 6,700 bp fragment,

### 5) Ligation of the BglII-BglII fragment

The plasmid resulting from (4) above was linearized with BglII. The 2.3 kb-BglII-BglII fragment was ligated together with the linearized plasmid. Bacterial colonies were analysed to find the resulting plasmid. The plasmid-DNA was digested with EcoRI and two resulting fragments were obtained, a 700 bp fragment (containing the promoter and a part of the HBV-sequence) and a 8,700 bp fragment (containing the rest of the HBV-sequence, MT-neo and plasmid).

### 6) Alterations within the HBV-sequence

The plasmid resulting from (5) above was digested with the endonucleases BglII and MstII. Two molecules were generated, one of 300 bp containing part of the pre-S sequence and the other (9,100 bp) which was eluted as described above. This 9,100 bp fragment was ligated to another BglII/MstII 216 bp fragment (sequence 
= AGATCTACAGCATGGGCAGAATCTTTCCACCAGCAATCCTCTGGGATTCTTTCCCGACCA
BglII S1
CCAGTTGGATCCAGCCTTCAGAGCAAACACCGCAAATCCAGATTGGGACTTCAATCCCAA
CAAGGACACCTGGCCAGACGCCAACAAGGTAGGAGCTGGAGCATTCGGCCTGGGTTTCAC
CCCACCGCACGGAGGCCTTTTGGGGTGGAGCCCTCAGG )
MstII
 coding for an altered pre-S₁ gene sequence.

The desired plasmid was digested with EcoRI and two resulting fragments were isolated, a 616 bp fragment and a 8,700 bp fragment.

### Example 5

### Isolation of the H2K Promoter

The H2K promoter was isolated as an EcoRI/BglII fragment (2kb) from psp65H2 (available from Exogene).

### Isolation of the egpt selection marker

The fragment containing the methallothionein promoter and the egpt-selection gene was isolated by digestion of the plasmid pMSG (available from Pharmacia) with the restriction enzyme EcoRI as a 3.6 kb fragment.

All other plasmid constructions were made in similar ways by combining fragments containing the necessary components and employing desired oligonucleotides and adapter sequences (where necessary).

### Example 6

### Transfection of Mammalian Cells with Constructs of the Present Invention.

In order to achieve secretion of substantial amounts of the HBV peptides encoded by constructs of the present invention, mammalian cells must be transfected with both the construct of the present invention and a construct which will express entire S protein. The cotransfection was performed in two steps (i.e., a separate transfection for each construct) or in a single step (i.e., one transfection using preparation of both constructs). Cotransfection was confirmed either by use of different selection markers on the two constructs or by detection of secretion of expression products of both constructs by immunoassay.

Alternatively, a sequence encoding the HBV peptide sequence of the present invention and a separate sequence encoding the entire S protein could be combined in a single construct.

### Example 7

### General Procedures

General procedures useful in practicing the present invention may be found in (1) Methods of Enzymology, volume 152, "Guide to Molecular Cloning Techniques," ed. Berger and Kimmel (Academic Press 1987), and (2) Maniatis et al., "Molecular Cloning: A Laboratory Manual," (Cold Spring Harber Laboratory 1982), both of which are incorporated herein in their antirety by reference. Specific techniques employed are described below.

### 1) Digestion with Endonucleases and Isolation of Fragments

The restriction endonucleases used were: BglII, BamHI, HindIII, EcoRI, XbaI, MstII, XhoI, Pf1MI, commercially available from Gibco/BRL with their respective restriction buffers (10x).

Unless otherwire indicated, restriction digests were performed and fragments were isolated as follows.
Reactions typically contained 1-5 ug DNA.
- distilled water was added to the DNA in an eppendorf tube to a final volume of 8 ul
- 1 ul of the appropriate 10x digestion buffer was added
- 1 ul (containing 5-10 U) restriction enzyme was added and mixed carefully
- the reaction tube was incubated for 1 hour at 37°C
- digestion was stopped by adding 0.5 M EDTA (pH 8.0) to a final concentration of 10 mM
- if the DNA was analysed directly on a gel, 1 ul of gel-loading dye III (Maniatis) was added, mixed and the sample was loaded into the slots of a 0.8% agarose gel.

The agarose gel normally contains 0.8% agarose 1% running buffer (TBE, Maniatis). Where a fragment (about 100-1000bp) was isolated from an agarose gel the agarose was increased to 1.2 to 1.4%.

### 2) Competent Bacterial Cells

From a dense overnight culture, 1 ml of the bacterial cell suspension was added to 100 ml fresh growth medium (L-broth). The cells were grown at 37°C to a density of OD₆₀₀ = 0.7 which was reached within 2 hours with vigorous shaking in a 500 ml Erlenmeyer flask. Growth was stopped by chilling the culture on ice for 10 minutes. From this culture, 3 ml were taken for harvesting the exponential bacterial cells at 3,000 rpm for 5 minutes. The cells were resuspended in 1.5 ml of 50 mM CaCl₂ in 10 mM Tris, pH 8.0, and incubated on ice for another 15 minutes. The cells were harvested once more by centrifugation at 3,000 rpm for 5 minutes and resuspended in 200 ul of 50 mM CaCl₂ in 10 mM Tris, pH 8.0, and used directly.

### 3) Transformation of Competent Bacterial Cells

The DNA to be transformed was suspended in 10 mM Tris, pH 7.5, 1 mM EDTA 70 ul and added to the 200 ul bacterial cell suspension for DNA take-up. The mixture was incubated on ice for 30 minutes and then 1 ml L-broth was added. The mixture was incubated at 42°C for 2 minutes and at 37°C for 40 minutes.
After the incubation, the cells were spread on agar plates containing 50 ug ampicillin/ml agar at volumes of 50-300 ul cell suspension per plate. The agar plates were incubated at 37°C overnight. After this incubation period, single isolated bacterial colonies were formed.

### 4) Plasmid DNA Isolation

1 liter of plasmid-bearing cells was grown to 0.5 OD₆₀₀ in L-broth and amplified for 20 hours with 200 ug/ml chloramphenicol. The culture was then centrifuged at 4,000 rpm for 20 minutes in JA-10 rotor, 4°C. The pellet was resuspended in 18 ml cold 25% sucrose, 50 mM Tris, pH 8.0 transferred to a 250 ml Erlenmeyer flask and kept on ice 6 ml 5mg/ml lysozyme in 250 mM Tris pH 8.0 was added and the mixture was left to stand 10-15 minutes. 6 ml 250 mM EDTA, pH 8.0, was added, mixed gently and incubated for 15 minutes on ice. 30 ml detergent (0.01% Triton X-100; 60 mM EDTA, pH 8.0; 50 mM Tris, pH 8.0) was added and the mixture was incubated for 30 minutes on ice. After incubation, the mixture was centrifuged at 25,000 rpm 90 minutes in SW28 rotor, 4°C.

Pronase was added to supernatant fluid to 250 ug/ml and incubated 30 minutes, 37°C. The solution was extracted with phenol once with 1/2 volume phenol equilibrated with 10 mM Tris, pH 8.0, 1 mM EDTA. The aqueous layer was removed. Sodium acetate was then added to a final concentration of 300 mM, followed by the addition of 3 volumes cold 100% ethanol and thorough mixing. The mixture was stored at -20°C overnight.

The mixture was thawed and centrifuged. The pellet was resuspended in 6 ml 10 mM Tris, 10 mM EDTA, pH 8.0. 9.4 g CsCl and 0.65 ml of 6 mg/ml ethidium bromide were added and the volume was brought up to 10 ml with sterile double-distilled water. The 10 ml alignots were put into Beckman heat-sealable gradient tubes and centrifuged, 50,000 rpm, 48 hours in Ti70.1 Beckman rotor.

Plasmid bands were visualized with UV and removed with syringe and 18 gauge needle by piercing the side of the tube. Ethidium bromide was removed from the plasmid fractions by 3 successive extractions with equal volumes of isobutanol. Fractions were then (1) dialyzed against one 2-liter lot of 10 mM Tris, pH 7.4, 1 mM EDTA, pH 7.5, 5 mM NaCl for 2 hours or more at 4°C; and (2) phenol extracted once with 1/3 volume phenol equilibrated as above. Sodium acetate was then added to a final concentration of 300 mM, followed by addition of two volumes of 100% ethanol. Precipitate formed at -20°C overnight, or at -70°C for 30 minutes.

### 5) Mini-Plasmid Preparation

1 ml of an overnight bacteria culture was put into an eppendorf tube and centrifugated for 20 minutes. The supernatant was removed. 100 ul of 50 mM glucose, 25 mM Tris (pH 8.0), 10 mM EDTA (pH 8.0) was added to the pellet, mixed by vortex and incubated for 5 minutes at room temperature. 200 ul of 0.2 N NaOH, 1% SDS was added, mixed by vortex and incubated for 5 minutes on ice. 150 ul 3 M Sodium acetate (pH 4.8) was added, mixed by vortex and incubated for 5 minutes on ice. After centrifugation for 5 minutes at 13,000 rpm the supernatant was decanted into a fresh eppendorf tube. 3 volumes of 100% ethanol were supplemented, mixed well and incubated for 30 minutes at -80°C, then centrifuged for 10 minutes at 13,000 rpm. The ethanol was removed, the pellet washed with 70% ethanol, lyophilized and dissolved in 20 ul distilled water. 5 ul of this plasmid DNA solution were used directly for restriction analysis.

### 6) Nick Translation

Nick translation was performed according to Rigby et al., J. Mol. Biol., Vol. 113, pp. 237-251, 1977, which is incorporated herein by reference. The reaction mixture for ³²P-labeling of DNA contained 0.5 ug of a HBV fragment, in a total volume of 30 ul with 50 mM Tris, pH 7.8, 5 mM MgCl₂, 10 mM mercaptoethanol, 0.1 mM dATP, 0.1 mM dGTP, 0.1 mM dTTP, 50 uCi ³²P-dCTP, 10 units DNA polymerase I, 3 ul of a 2 x 10⁻⁵ fold dilution of 1 mg/ml DNase I and is incubated for 90 minutes at 15°C, yielding 3 x 10⁶ to 12 x 10⁶ total cpm, i.e. 1 x 10⁷ to 5 x 10⁷ cpm/ug DNA.

### 7) Southern Blot Analysis

To characterize the organization within the host cell genome of the vectors of this invention, chromosomal DNA from cell lines producing particles of this invention were isolated and digested with the appropriate restriction enzyme(s) and analysed by the method of Southern (J. Mol. Biol., Vol. 98, pp. 503-517, 1975), which is incorporated herein by reference, using a ³²P-labeled DNA probe. Following digestion of the chromosomal DNA (20 µg) with the restriction enzyme BglII, the resulting fragments were separated by 0.7% agarose gel electrophoresis. Thereafter, the DNA was denatured by exposing to 366 nm UV light for 10 minutes and by incubation in a solution of 0.5 M NaOH and 1 M NaCl for 45 minutes. The gels were neutralized by incubation in 0.5 M Tris, 1.5 M NaCl, pH 7.5 for 60 minutes. The DNA was transferred to a nitrocellulose filter by soaking in 3 M NaCl, 0.3 M Sodiumcitrate (20 x SSC) for 20 hours through the gel by covering the top of the nitrocellulose filter with a staple of dry paper towels. The nitrocellulose filter was kept for 2 hours in a vacuum oven at 80 C. A radioactive DNA probe from the BglII fragment of the pHBV (2.3 kb) was prepared by nick translation.
For hybridization with the DNA probe, the nitrocellulose filter was sealed in a plastic bag containing 10 ml of prehybridization mixture: 50% formamide, 5 x SSC, 50 mM Sodiumphosphate, pH 7.0, 5 x Denhardt's solution, 250 ug/ml denatured salmon sperm DNA. The filter was incubated in this mixture for 4 hours at 45°C, after which the pre-hybridization mixture was replaced by the hybridization mixture: 50% formamide, 5 x SSC, 20 mM Sodiumphosphate, pH 7.0, 1 x Denhardt's solution, 100 ug/ml denatured salmon sperm DNA, 5 x 10⁵ cmp/ml ³²P-probe, The filter, after incubating in the hybridization mix for 18 hours at 45°C, was washed three times, 5 minutes each, in 0.1 x SSC, 0.1% SDS at 50°C. The filter was dried at 60°C for 10 minutes and exposed to two X-ray films (XAR-5, KODAK) between two intensifying screens and kept at -80°C. The first X-ray film is developed after 3 days' exposure; the second film after 7 days' exposure.

### 8) Preparation of Mammalian Cells and DNA Precipitate for Transfection

The recipient cells (C127 or CHO-cells available from ATCC)were seeded in normal growth medium (DMEM+10% Fetal Calf Serum,Glycose and Glutamin) into petri-dishes (1-2 x 10⁶ cells per dish, 10 cm) at day 1. The next day the medium was removed (4 hours before the DNA precipitate was added onto the cells), and the cells were washed twice with 1 x PBS. Then 8 ml DMEM without FCS were added. 4 hours later the DNA precipitate (prepared as described below) was added to the cells. Again after 4 hours the medium was removed, 3 ml of Glycerol-Mix (50 ml 2 x TBS buffer, 30 ml glycerol, 120 ml distilled water) were added. The Glycerol-Mix was immediately removed after an incubation at 37°C for 3 minutes and the cells were washed with 1 x PBS. The cells were cultivated overnight with 8 ml of DMEM with 10% FCS.

After 48 hours, the cells were recovered from the dish by treating with Trypsin-EDTA-Solution (0.025% Trypsin + 1 mM EDTA). Afterwards, to remove the Trypsin-EDTA the cells were washed with 1 x PBS, suspended in DMEM with 10% FCS and distributed into 24 costar-well-plates (cells from one dish into four 24-well-plates). When the cells had grown well, selection medium was added (concentration 0.5 - 1mg/ml of neomycin,or xanthine: 250 µg/ml, hypoxanthine: 15 µg/ml (or adenine: 25 µg/ml), thymidine: 10 µg/ml,aminopterine 2 µg/ml mycophenolic acid: 25 µg/ml for eco-gpt, for example). The medium was changed every week. The first growing cell colonies were seen after 2 weeks.

To 10 ug of plasmid DNA and 20 ug of carrier-DNA (salmon-sperm DNA, calf-thymus DNA) TE-buffer (10 mM Trix-HCl, 1 mM EDTA, pH 7.05) was added to a final volume of 440 ul and mixed together with 60 ul 2 M CaCl₂. Then the same amount of 2x TBS (Hepes 50 mM, NaCl 280 mM, Na₂HPO₄ 1.5 mM, pH 7.05) was added and mixed well. The precipitation solution was incubated for 30 minutes at 37°C and added directly to the cells which should be transfected.

### Example 8

### Culturing of Transfected Cells to Secrete Protein

The selected cells are treated for further cultivation in normal growth medium as described in section 8.

### Example 9

### F) preparation of the Adjuvant of Purified Particles

To the desired concentration of antigen particles suspended in sterile saline, 1 ; 10,000 volume Thimerosol, 1/10 volume of filter-sterilized 0.2 M Al K(S04)₂ : 12 H₂0 were added. The pH was adjusted to 5.0 with sterile 1 N NaOH and the suspension was stirred at room temperature for 3 hours. The alum-precipitated antigen was recovered by centrifugation for 10 minutes at 2,000 rpm, resuspended in sterile normal saline containing 1:10,000 Thimerosol and aliquated under sterile conditions,

### Example 10

Tables III - X give some of the results of ELISA analysis of immunogenic particles as described below:
- Table III:: shows the ELISA data of the purified HBs antigen particle produced from any HBV sequence construct of the present invention including the pre-S₁ region with total deletion of pre-S₂ and deletions upstream of the pre-S₂ ATG and the S region with deletion of the S ATG and downstream the S ATG through the XBaI site (e.g. the construct of Fig. I-1) with the anti-pre-S₁ monoclonal antibody MA 18/7. The fractions 9-15 (Fig. XI) were pooled after CsCl sedimentation.
- Table IV:: shows the ELISA data of the purified HBS antigen particle produced from any HBV sequence construct of the present invention including the pre-S₁ region with total deletion of pre-S₂ and deletions upstream of the pre-S₂ ATG and the S region with deletion of the S ATG and downstream the S ATG through the XBaI site (e.g., the construct of Fig. I-1) with the anti-pre-S₂ monoclonal antibody MQ 19/10. The fractions 9-15 (Fig. XI) were pooled after CsCl sedimentation.
- Table V:: shows the ELISA data of the purified HBs antigen particle produced from an HBV sequence construct including the pre-S₂ region with none of the pre-S₁ region and deletions upstream of the S ATG and downstream of the S ATG through the XBaI site, and the S region with deletion of the S ATG (e.g. the construct of Fig. II-1) with the anti-pre-S₁ monoclonal antibody MA 18/7. The fractions 9-15 (Fig. XII) were pooled after CsCl sedimentation.
- Table VI:: shows the ELISA data of the purified HBS antigen particle produced from an HBV sequence construct including the pre-S₂ region with none of the pre-S₁ region and deletions upstream of the S ATG and downstream of the S ATG through the XBaI site, and the S region with deletion of the S ATG (e.g. the construct of Fig. II-1) with the anti-pre-S₂ monoclonal antibody MQ 19/10.
The fractions 9-15 (Fig. XII) were pooled after CsCl sedimentation.

**Table III**

| | |
|---|---|
| CsCl-gradient | ELISA Measurement |
| | Monoclonal Antibody MA 18/7 |
| Fraction No. 9-15 (pooled) | E₄₉₂ = 0.839 |

**Table IV**

| | |
|---|---|
| CsCl-gradient | ELISA Measurement |
| | Monoclonal Antibody MQ 19/10 |
| Fraction No. 9-15 (pooled) | E₄₉₂ = 0.000 |

**Table V**

| | |
|---|---|
| CsCl-gradient | ELISA Measurement |
| | Monoclonal Antibody MA 18/7 |
| Fraction No. 9-15 (pooled) | E₄₉₂ = 0.000 |

**Table VI**

| | |
|---|---|
| CsCl-gradient | ELISA Measurement |
| | Monoclonal Antibody MQ 19/10 |
| Fraction No. 9-15 (pooled) | E₄₉₂ = 1.028 |

Table VII: shows the ELISA data of the purified HBs antigen particle produced from any HBV sequence construct of the present invention including the pre-S₁ region with total deletion of pre-S₂ and deletions upstream of the pre-S₂ ATG and the S region with deletion of the S ATG (e.g., the construct of Fig. VI-2) with the anti-pre-S₁ monoclonal antibody MA 18/7. The fractions 9-15 (Fig. XI) were pooled after CsCl sedimentation.
Table VIII: shows the ELISA data of the purified HBs antigen particle produced from any HBV sequence construct of the present invention including the pre-S₁ region with deletions upstream of the pre-S₂ ATG with deletion of the S ATG with the anti-pre-S₂ monoclonal antibody MQ 19/10. The fractions 9-15' (Fig. XI) were pooled after CsCl sedimentation.
Table IX: shows the ELISA data of the purified HBs antigen particle produced from an HBV sequence construct including the pre-S₂ region with none of the pre-S₁ region and deletions upstream of the S ATG and the S region with deletion of the S ATG (e.g., the construct of Fig. VII-2) with the anti-pre-S₁ monoclonal antibody MA 18/7. The fractions 9-15 (Fig. XII) were pooled after CsCl sedimentation.
Table X: shows the ELISA data of the purified HBs antigen particle produced from an HBV sequence construct including the pre-S₂ region with deletions upstream of the S ATG with deletion of the S ATG (e.g., the construct of Fig. VII-4) with the anti-pre-S₂ monoclonal antibody MQ 19/10. The fractions 9-15 (Fig. XII) were pooled after CsCl sedimentation.

**Table VII**

| | |
|---|---|
| CsCl-gradient | ELISA Measurement |
| | Monoclonal Antibody MA 18/7 |
| Fraction No. 9-15 (pooled) | E₄₉₂ = 1.273 |

**Table VIII**

| | |
|---|---|
| CsCl-gradient | ELISA Measurement |
| | Monoclonal Antibody MQ 19/10 |
| Fraction No. 9-15 (pooled) | E₄₉₂ = 0.000 |

**Table IX**

| | |
|---|---|
| CsCl-gradient | ELISA Measurement |
| | Monoclonal Antibody MA 18/7 |
| Fraction No. 9-15 (pooled) | E₄₉₂ = 0.000 |

**Table X**

| | |
|---|---|
| CsCl-gradient | ELISA Measurement |
| | Monoclonal Antibody MQ 19/10 |
| Fraction No. 9-15 (pooled) | E₄₉₂ = 0.985 |

Table XI shows the ELISA data of purified HBs antigen particles produced by construct including the entire pre-S₁ - pre-S₂ - S region under control of the LTR region of rous sarcoma virus after stimulation with stimulating substances (e.g. PMA) and the additional cotransfection with S (Fig. XIII).

**Table XI**

| | |
|---|---|
| CsCl-gradient | ELISA Measurement |
| | Monoclonal Antibody MA 18/7 |
| Fraction No. 9-15 (pooled) | E₄₉₂ = 0.125 |

Figure XIV shows the characterisation of the particles derived from gene constructs according to table III (Fig. I-1) and table V (Fig. II-1) cotransfected in C127 after purification in the CsCl gradient. The fraction collected had a smaller volume.
Table XII shows the serotyping of particles according to Fig. I-1 having the S sequence done in the Pettenkofer Institute.

**Table XII**

| Results: | |
|---|---|
| adw / ayw | positive |

From the foregoing, it will be obvious to those skilled in the art that various modifications in the above-described compositions and methods can be made without departing from the spirit and scope of the invention. Accordingly, the invention may be embodied in other specific forms without departing from the spirit or essential characteristics thereof. Present embodiments, therefore, are to be considered in all respects as illustrative and not restrictive, the scope of the invention being indicated by the appended claims rather than by the foregoing description, and all changes which come within the meaning and range of equivalency of the claims are therefore intended to be embraced therein.

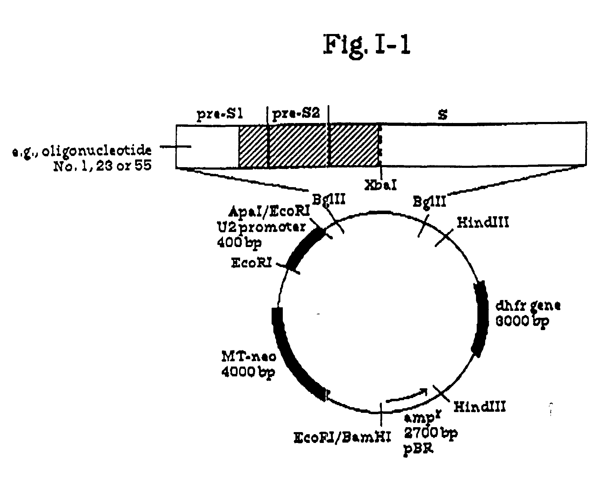

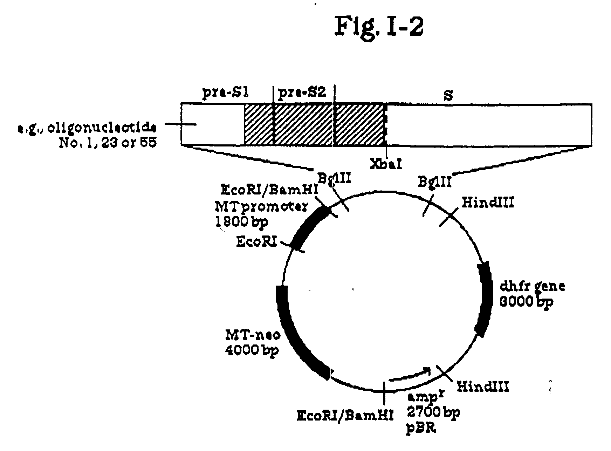

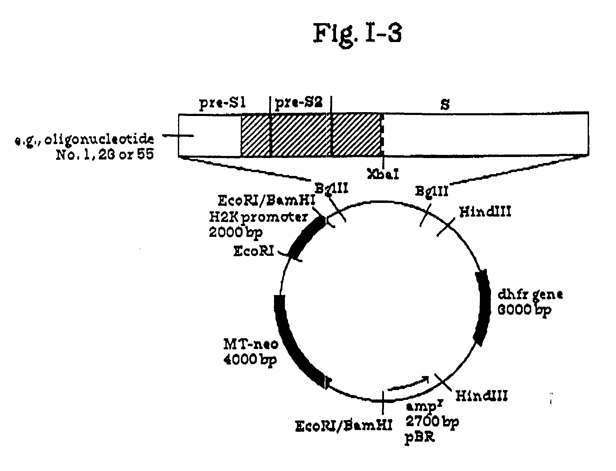

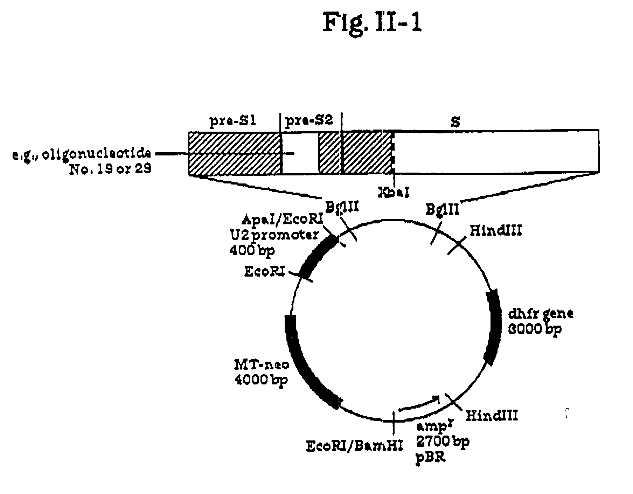

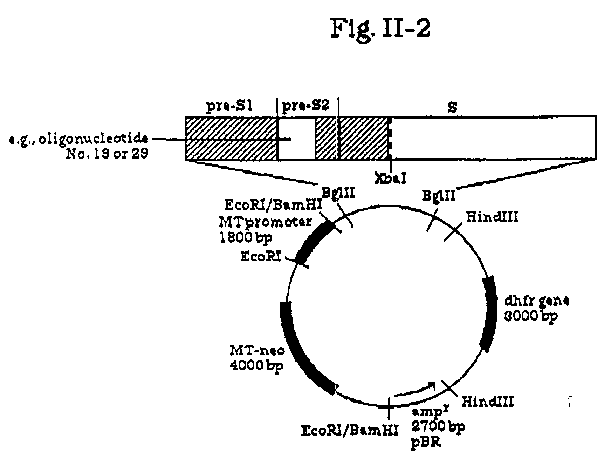

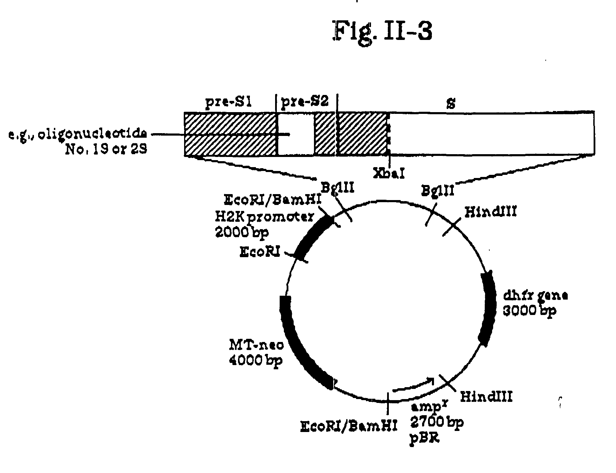

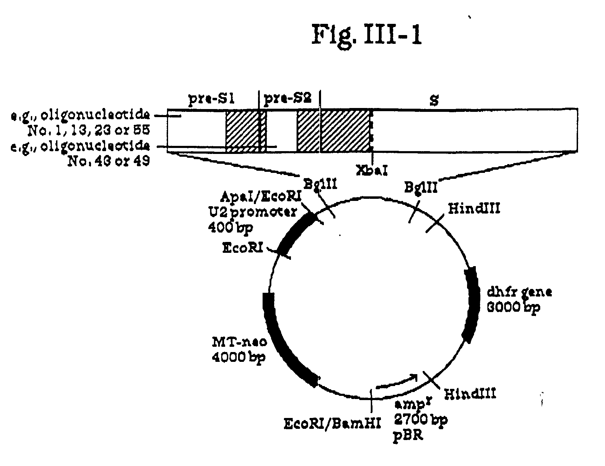

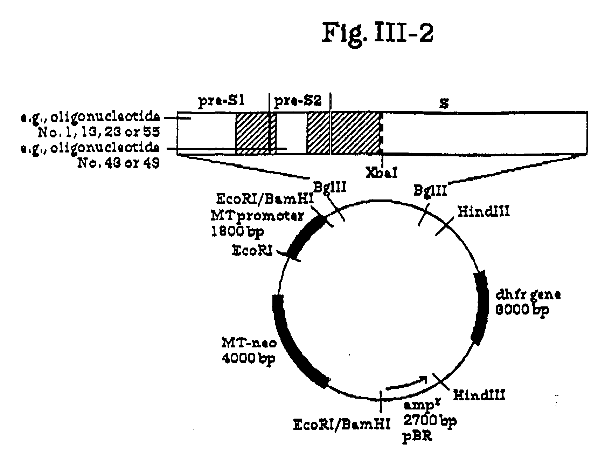

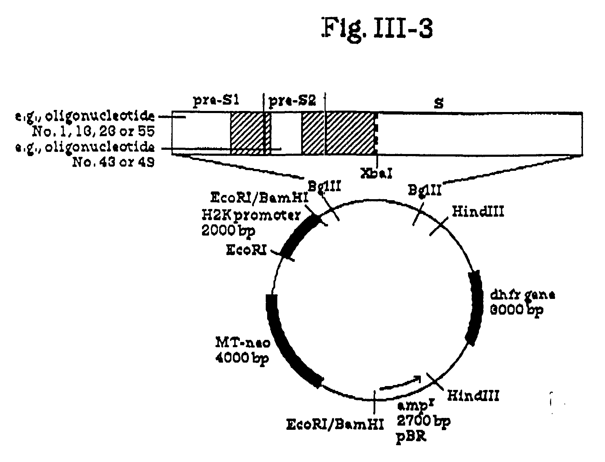

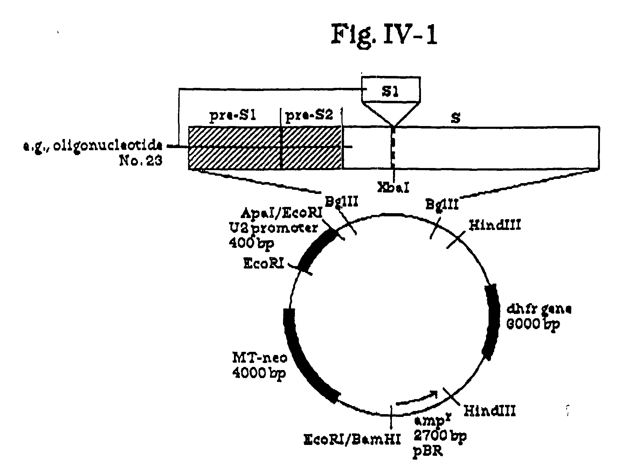

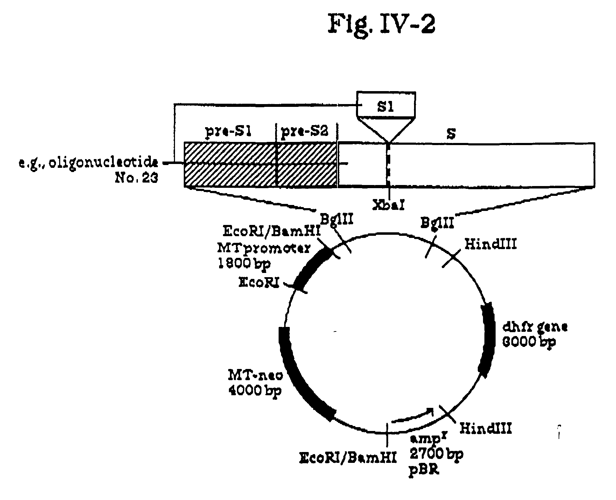

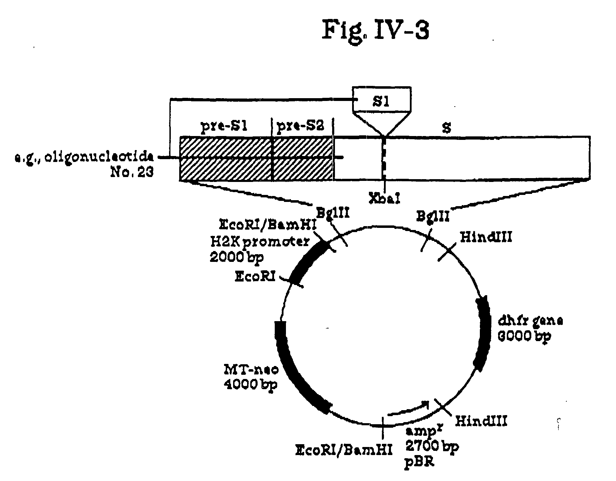

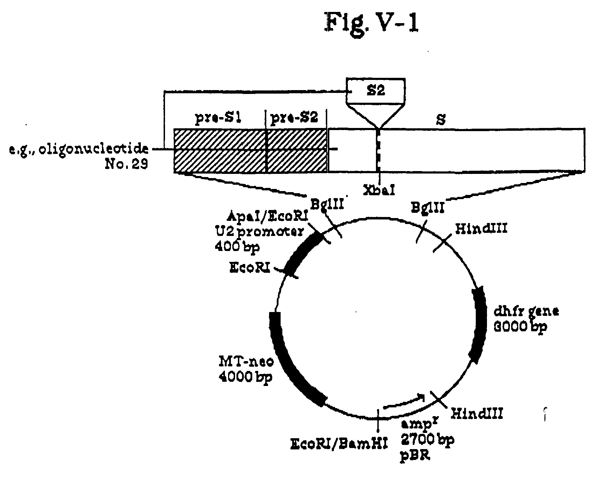

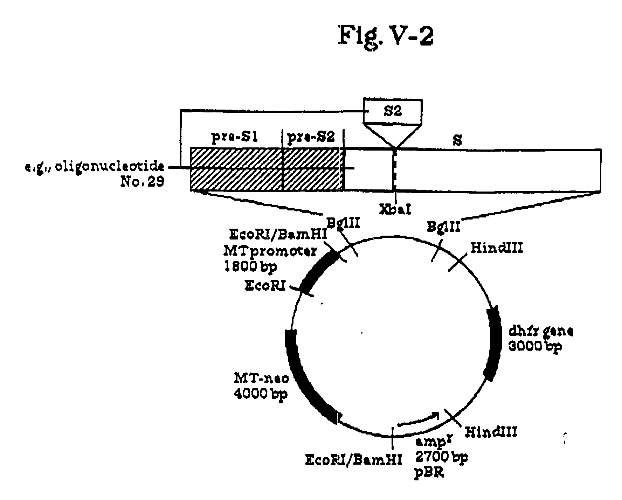

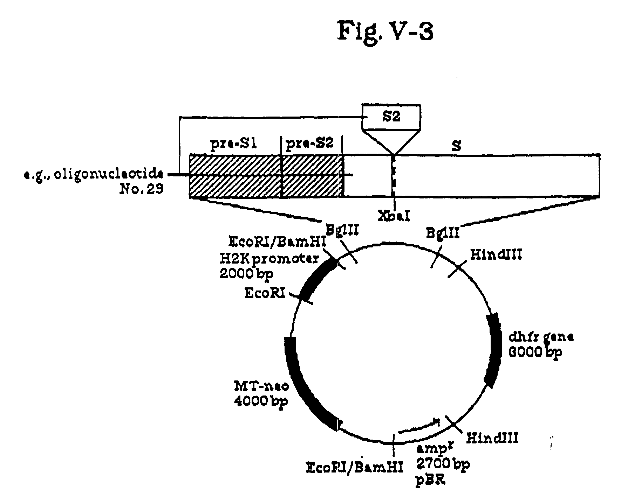

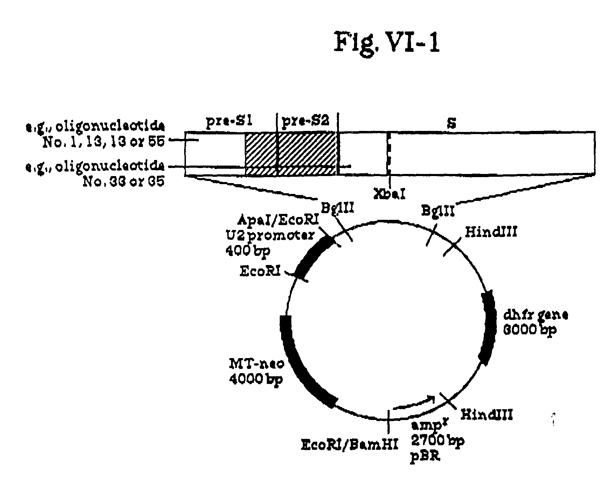

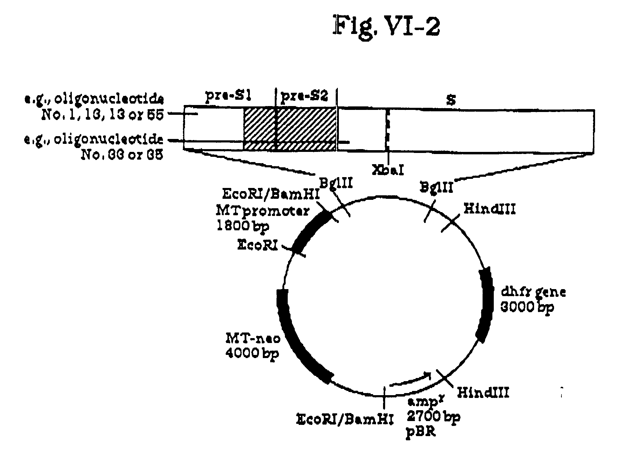

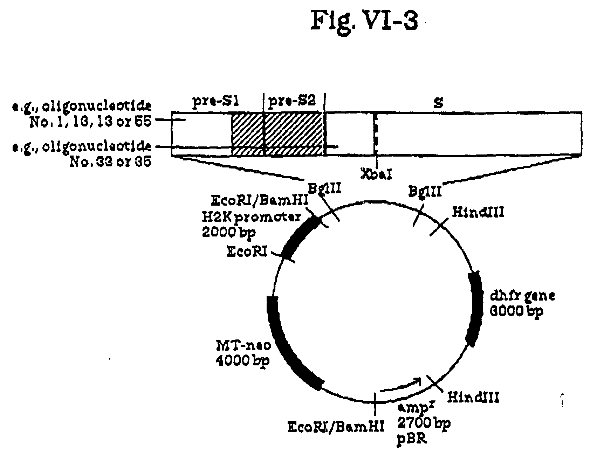

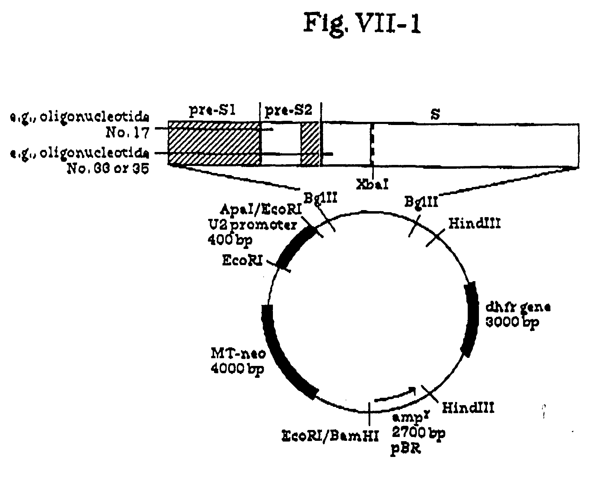

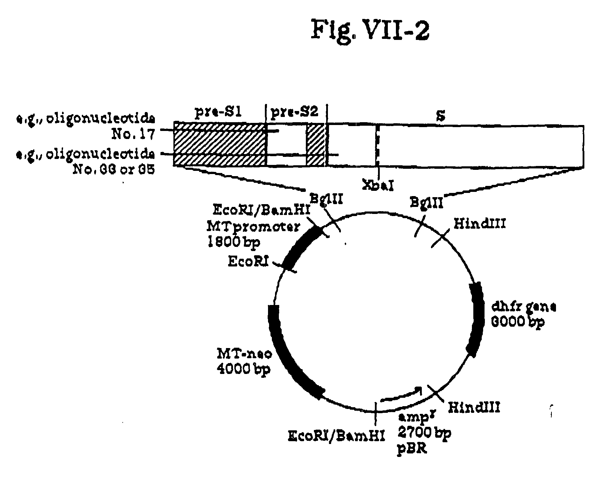

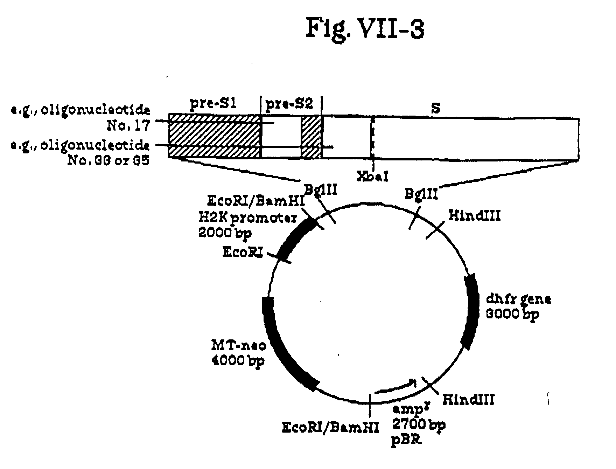

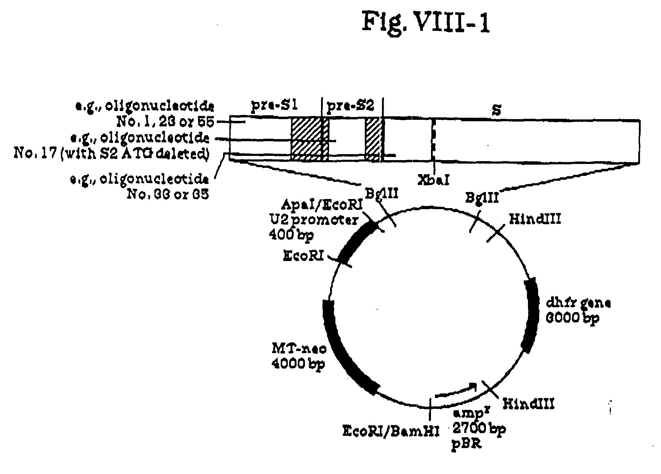

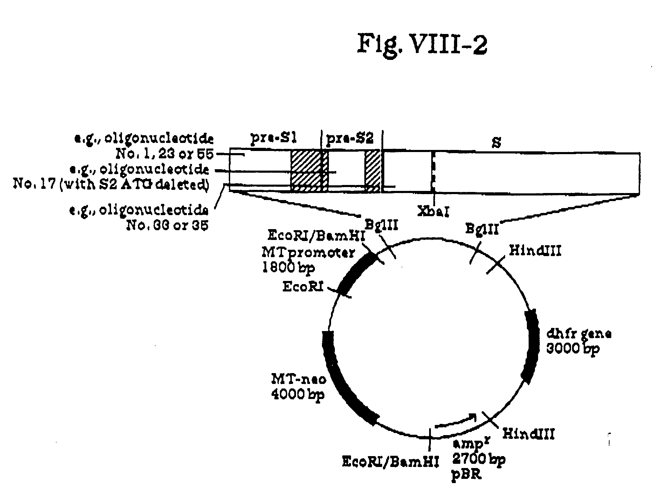

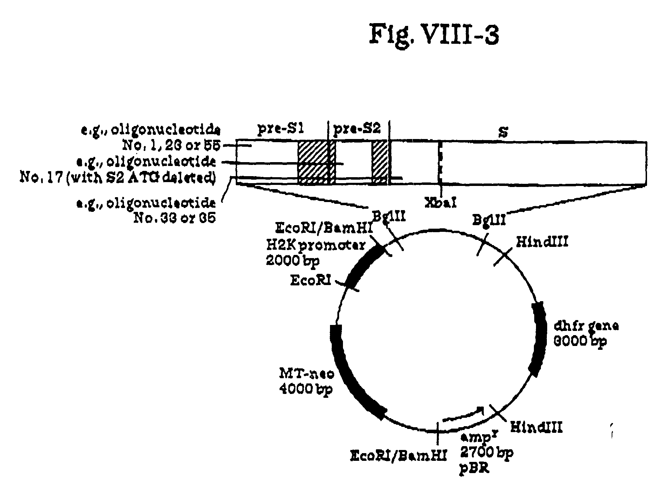

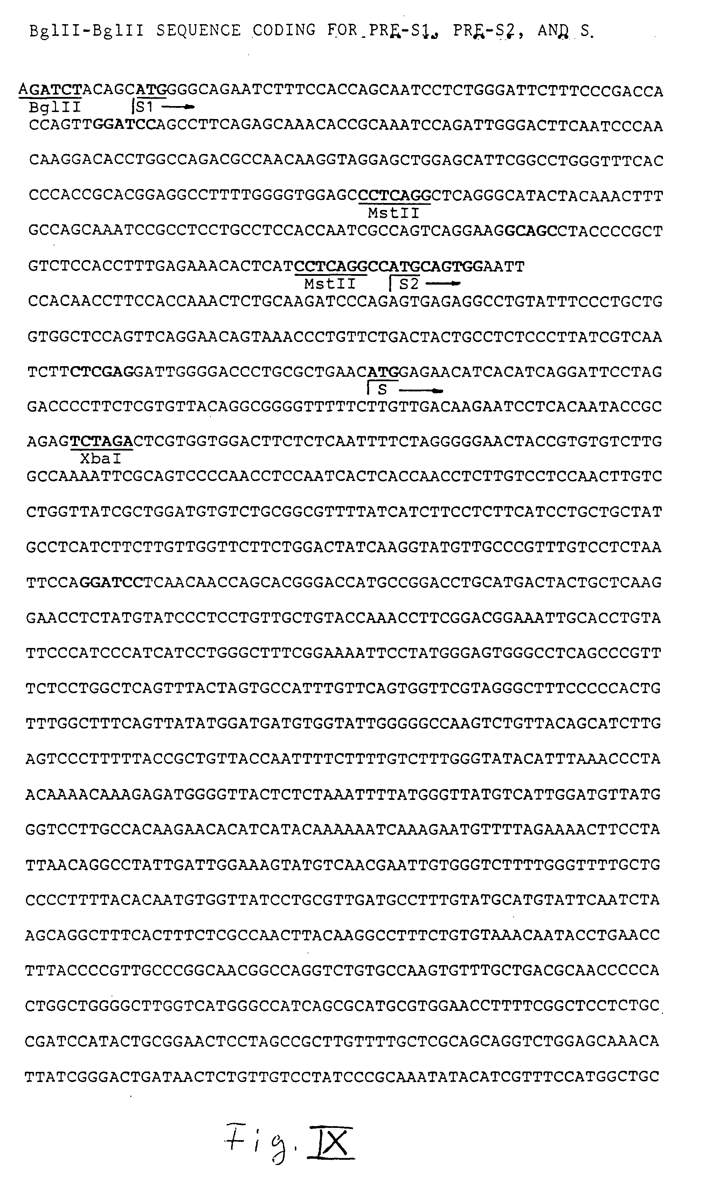

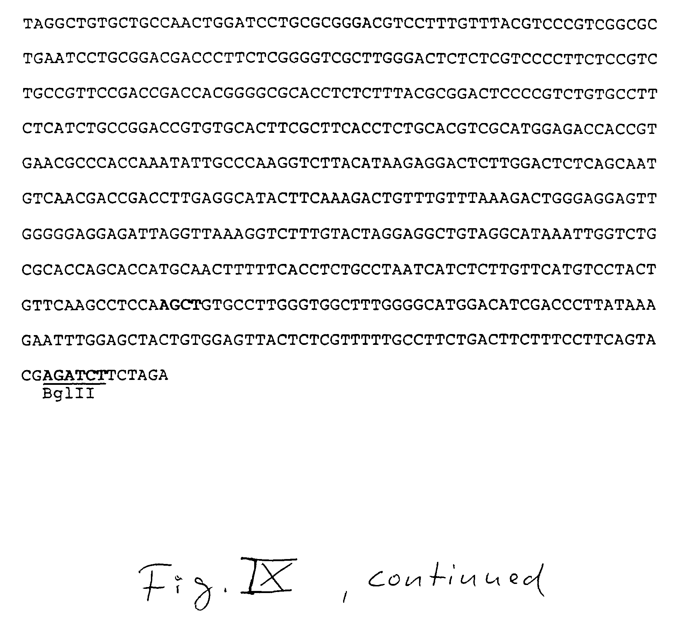

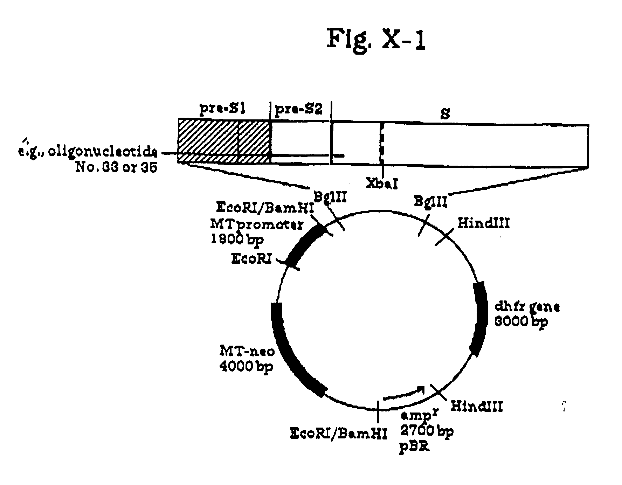

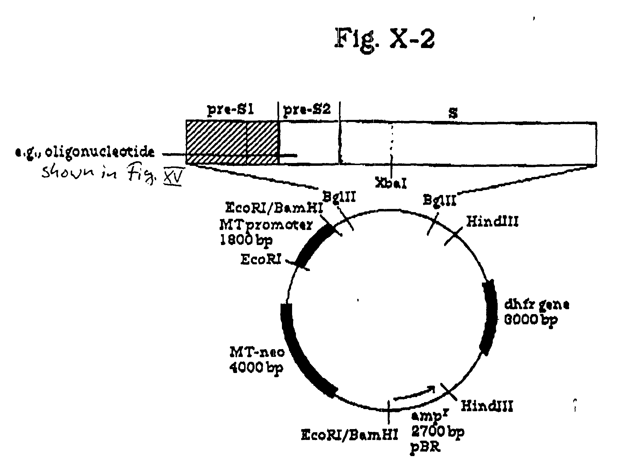

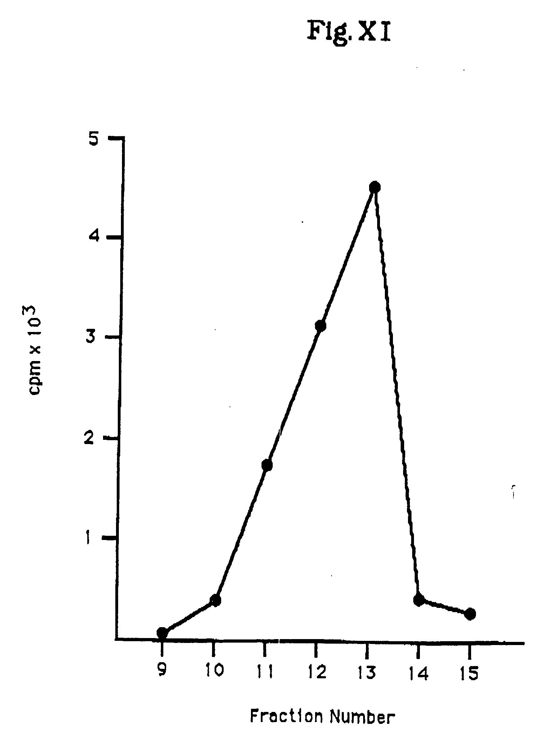

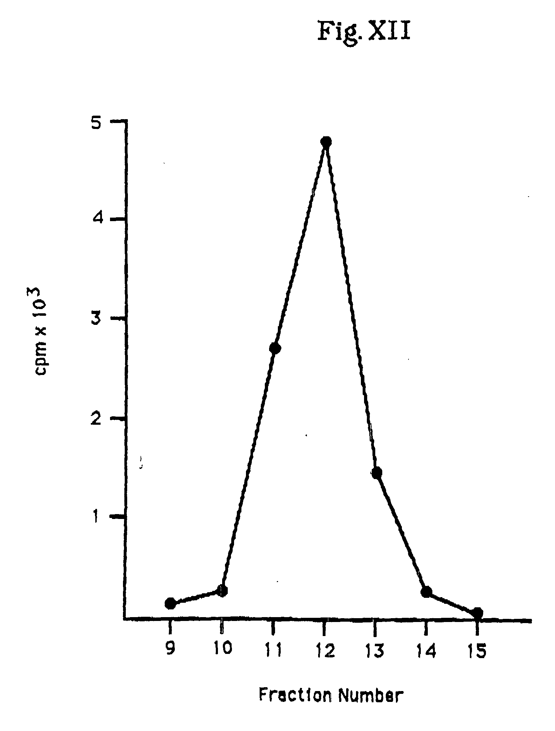

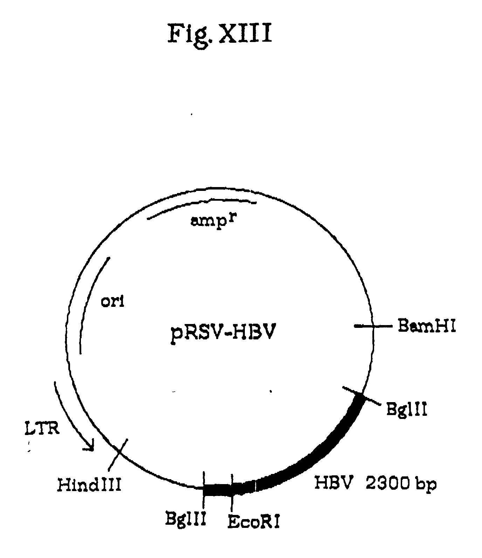

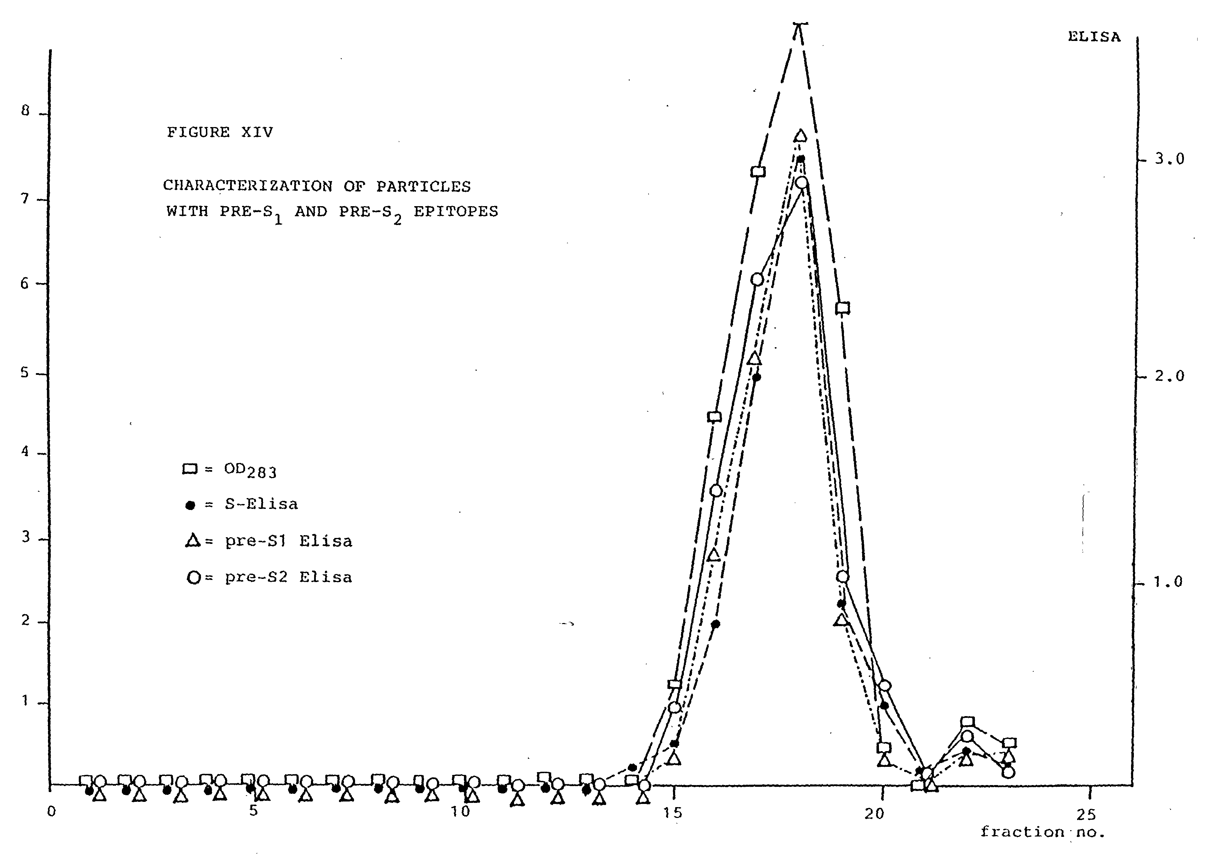

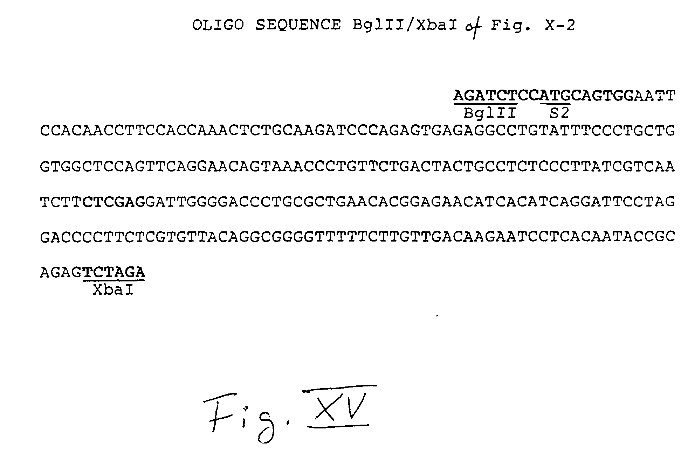

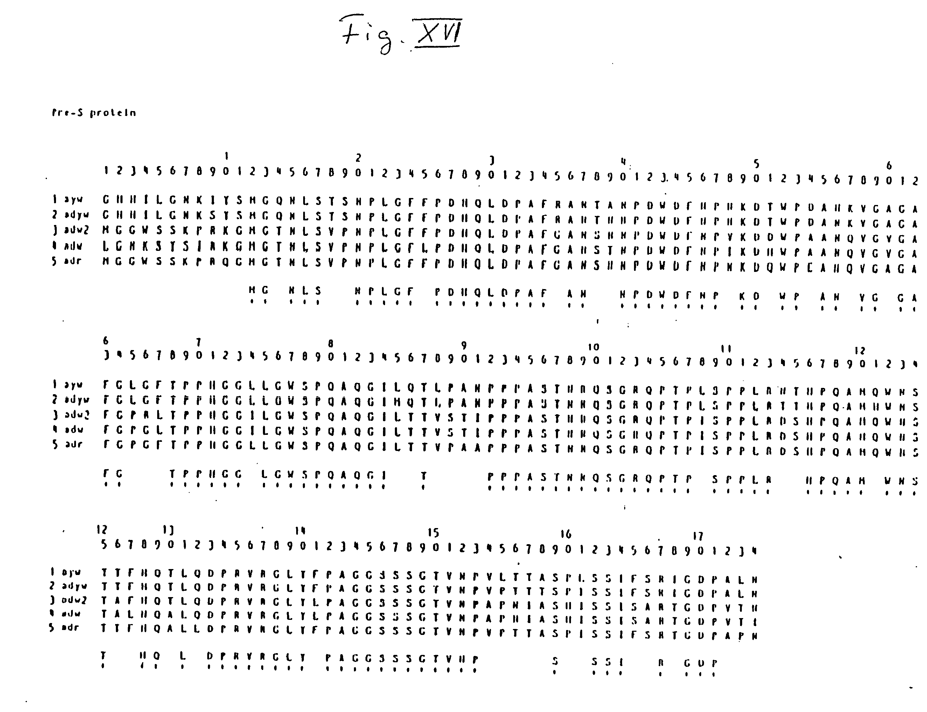

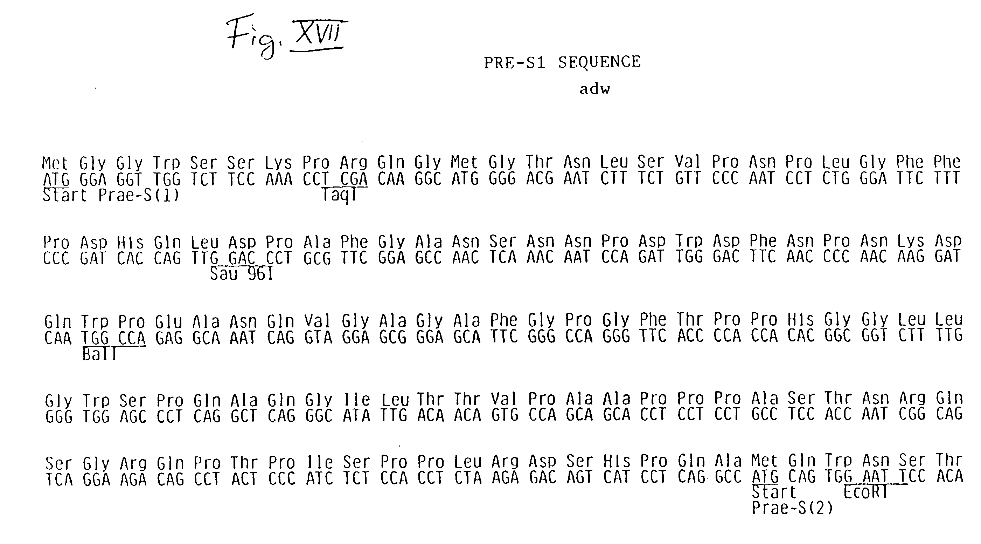

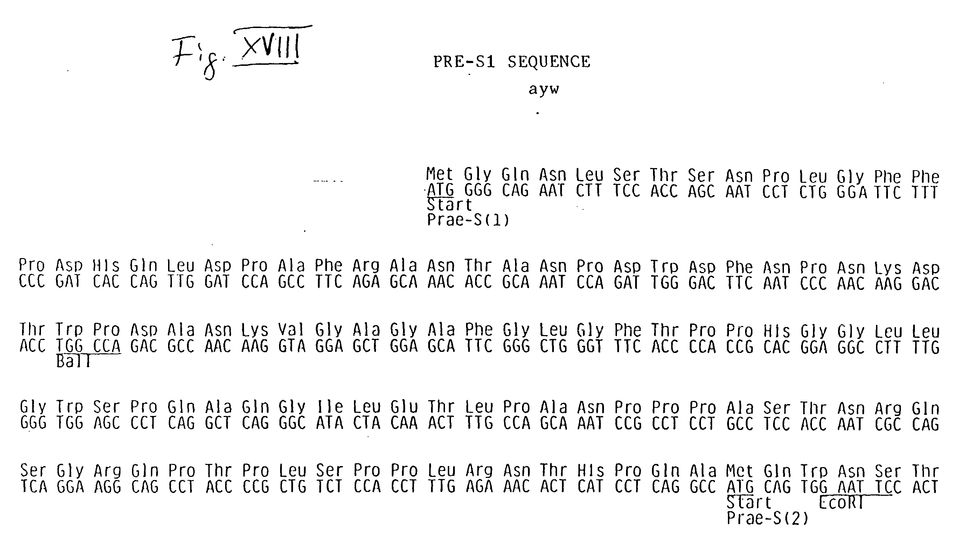

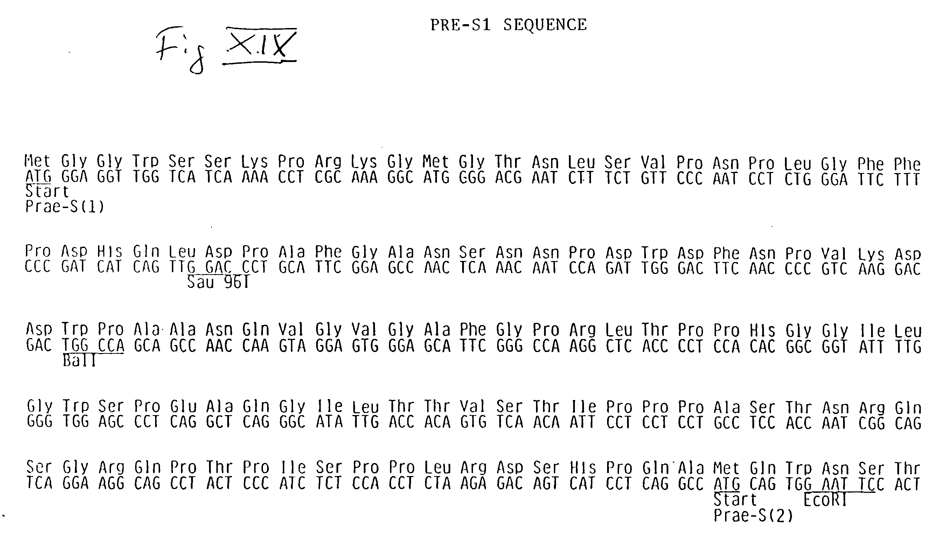

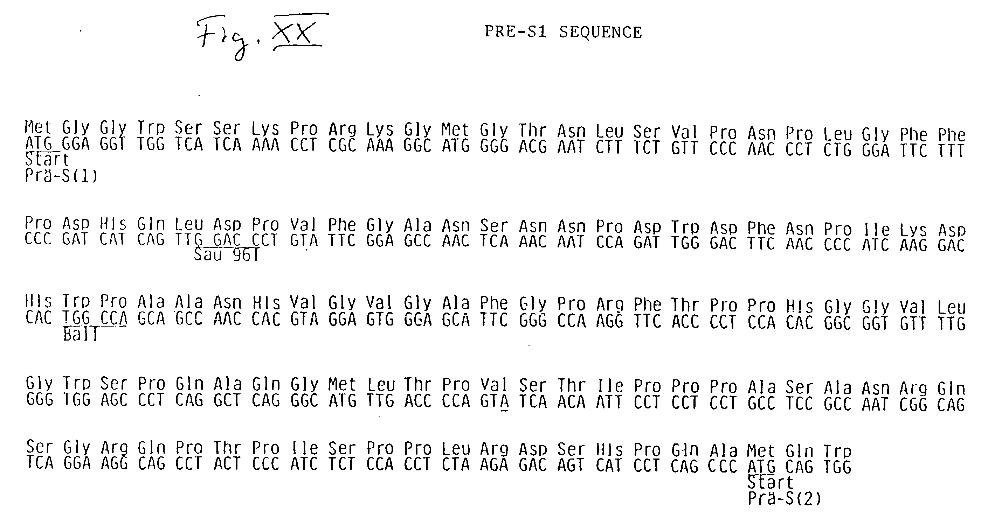

## Claims (Claims for the following Contracting State(s): AT, BE, CH, LI, DE, FR, GB, IT, LU, NL and SE)

1. A recombinant DNA molecule comprising an expression control sequence operatively linked to first and second DNA sequences linked in frame, said DNA sequences each encoding a discrete region of a single peptide expressed by said recombinant DNA molecule, and said first DNA sequence being closer to said expression control sequence than said second DNA sequence, **characterised in that**:
(a) said first DNA sequence comprises the nucleotide sequence of part of the HBV pre-S₁ coding region wherein a 5'- terminal region of said pre-S₁ coding region containing the pre-S₁ region ATG start codon has been replaced by a 5'- terminal coding region of the HBV S region containing the S region ATG start codon, and said first DNA sequence encodes an HBV pre-S₁ epitope; and
(b) said second DNA sequence comprises nucleotide sequence of the HBV S coding region wherein the ATG start codon is absent and encodes a peptide which upon secretion will direct formation of particles that will provoke an immune response against the pre-S₁ epitope.

2. A DNA molecule as claimed in claim 1 in which the nucleotide sequence of said 5'- terminal coding region of the HBV S region is ATGGAGAAC.

3. A DNA molecule as claimed in claim 2 in which the nucleotide sequence of said 5'- terminal coding region of the HBV S region is AACATGGAGAAC.

4. A DNA molecule as claimed in any one of claims 1 to 3 wherein said first DNA sequence comprises the nucleotide sequence of part of the HBV pre-S₁ coding region wherein the 3'- terminus of the pre-S₁ coding region has been deleted.

5. A DNA molecule as claimed in any of claims 1 to 4 coding for an HBV pre-S₁ epitope of the ayw serotype.

6. A DNA molecule as claimed in any of claims 1 to 5 containing at least one nucleotide sequence from Table I.

7. A DNA molecule as claimed in claim 6 wherein said first DNA sequence comprises the sequence of oligo no. 55 from Table I.

8. A DNA molecule as claimed in claim 6 wherein said first DNA sequence comprises the sequence of oligo no. 23 from Table I.

9. A DNA molecule as claimed in claim 7 or claim 8 in which said second DNA sequence comprises the S-coding sequence of the XbaI-BglII fragment of the HBV S gene shown in Figure IX.

10. A DNA molecule as claimed in claim 1, said DNA molecule being described by Figure I-1, I-2, IV-1, IV-2, IV-3, VI-1, VI-2 or VI-3.

11. A host cell transfected with a recombinant DNA molecule as claimed in any of claims 1 to 10.

12. A host cell co-transfected with a first recombinant DNA molecule and a second recombinant DNA molecule, said first recombinant DNA molecule being a recombinant DNA molecule as claimed in any one of claims 1 to 10 and said second recombinant DNA molecule encoding a peptide including the amino acid sequence of the HBV S peptide.

13. A host cell as claimed in claim 11 or 12 which is of eukaryotic origin.

14. A host cell as claimed in claim 13 which is a yeast cell.

15. A host cell as claimed in claim 13 which is of mammalian origin.

16. A host cell as claimed in claim 15 which is selected from a VERO cell, a 3T3 cell, a C127 cell, an L cell, a CHO cell which is positive for dehydrofolate reductase and a CHO cell which is negative for dehydrofolate reducatase.

17. A peptide encoded by a recombinant DNA molecule as claimed in any of claims 1 to 10.

18. A peptide as claimed in claim 17 for use in a method of vaccination against HBV.

19. A process for producing a transfected host cell, said process comprising:
providing a host cell which has been made competent for uptake of DNA,
exposing said host cell to a first preparation of DNA comprising a recombinant DNA molecule of any of claims 1 to 10,
allowing under suitable conditions said host cell to take up DNA from said first preparation of DNA, and selecting for a host cell which has taken up said recombinant DNA molecule.

20. A process as claimed in claim 19, further comprising exposing said host cell to a second preparation of DNA comprising a DNA molecule encoding a peptide including the amino acid sequence of the HBV S peptide, and allowing under suitable conditions said host cell to take up DNA from the second preparation of DNA.

21. A method of producing a peptide, said method comprising culturing a host cell transfected with a recombinant DNA molecule as claimed in any of claims 1 to 10 under conditions allowing expression and secretion of protein by said cell, and collecting the peptide produced as a result of expression from said recombinant DNA molecule.

## Claims (Claims for the following Contracting State(s): ES AND GR)

1. A process for producing a transfected host cell, said process comprising:
providing a host cell which has been made competent for uptake of DNA,
exposing said host cell to a first preparation of DNA comprising a recombinant DNA molecule,
allowing under suitable conditions said host cell to take up DNA from said first preparation of DNA, and
selecting for a host cell which has taken up said recombinant DNA molecule,
wherein said recombinant DNA molecule comprises an expression control sequence operatively linked to first and second DNA sequences linked in frame, said DNA sequences each encoding a discrete region of a single peptide expressed by said recombinant DNA molecule, and said first DNA sequence being closer to said expression control sequence than said second DNA sequence, **characterised in that**:
(a) said first DNA sequence comprises the nucleotide sequence of part of the HBV pre-S₁ coding region wherein a 5'- terminal region of said pre-S₁ coding region containing the pre-S₁ region ATG start codon has been replaced by a 5'- terminal coding region of the HBV S region containing the S region ATG start codon, and said first DNA sequence encodes an HBV pre-S₁ epitope; and
(b) said second DNA sequence comprises nucleotide sequence of the HBV S coding region wherein the ATG start codon is absent and encodes a peptide which upon secretion will direct formation of particles that will provoke an immune response against the pre-S₁ epitope.

2. A process as claimed in claim 1, further comprising exposing said host cell to a second preparation of DNA comprising a DNA molecule encoding a peptide including the amino acid sequence of the HBV S peptide, and allowing under suitable conditions said host cell to take up DNA from the second preparation of DNA.

3. A method of producing a peptide, said method comprising culturing a host cell transfected with a recombinant DNA molecule under conditions allowing expression and secretion of protein by said cell, and collecting the peptide produced as a result of expression from said recombinant DNA molecule, wherein said recombinant DNA molecule comprises an expression control sequence operatively linked to first and second DNA sequences linked in frame, said DNA sequences each encoding a discrete region of a single peptide expressed by said recombinant DNA molecule, and said first DNA sequence being closer to said expression control sequence than said second DNA sequence, **characterised in that**:
(a) said first DNA sequence comprises the nucleotide sequence of part of the HBV pre-S₁ coding region wherein a 5'- terminal region of said pre-S₁ coding region containing the pre-S₁ region ATG start codon has been replaced by a 5'- terminal coding region of the HBV S region containing the S region ATG start codon, and said first DNA sequence encodes an HBV pre-S₁ epitope; and
(b) said second DNA sequence comprises nucleotide sequence of the HBV S coding region wherein the ATG start codon is absent and encodes a peptide which upon secretion will direct formation of particles that will provoke an immune response against the pre-S₁ epitope.

4. A recombinant DNA molecule comprising an expression control sequence operatively linked to first and second DNA sequences linked in frame, said DNA sequences each encoding a discrete region of a single peptide expressed by said recombinant DNA molecule, and said first DNA sequence being closer to said expression control sequence than said second DNA sequence, **characterised in that**:
(a) said first DNA sequence comprises the nucleotide sequence of part of the HBV pre-S₁ coding region wherein a 5'- terminal region of said pre-S₁ coding region containing the pre-S₁ region ATG start codon has been replaced by a 5'- terminal coding region of the HBV S region containing the S region ATG start codon, and said first DNA sequence encodes an HBV pre-S₁ epitope; and
(b) said second DNA sequence comprises nucleotide sequence of the HBV S coding region wherein the ATG start codon is absent and encodes a peptide which upon secretion will direct formation of particles that will provoke an immune response against the pre-S₁ epitope.

5. A DNA molecule as claimed in claim 4 in which the nucleotide sequence of said 5'- terminal coding region of the HBV S region is ATGGAGAAC.

6. A DNA molecule as claimed in claim 5 in which the nucleotide sequence of said 5'- terminal coding region of the HBV S region is AACATGGAGAAC.

7. A DNA molecule as claimed in any one of claims 4 to 6 wherein said first DNA sequence comprises the nucleotide sequence of part of the HBV pre-S₁ coding region wherein the 3'- terminus of the pre-S₁ coding region has been deleted.

8. A DNA molecule as claimed in any of claims 4 to 7 coding for an HBV pre-S₁ epitope of the ayw serotype.

9. A DNA molecule as claimed in any of claims 4 to 8 containing at least one nucleotide sequence from Table I.

10. A DNA molecule as claimed in claim 9 wherein said first DNA sequence comprises the sequence of oligo no. 55 from Table I.

11. A DNA molecule as claimed in claim 9 wherein said first DNA sequence comprises the sequence of oligo no. 23 from Table I.

12. A DNA molecule as claimed in claim 10 or claim 11 in which said second DNA sequence comprises the S-coding sequence of the XbaI-BglII fragment of the HBV S gene shown in Figure IX.

13. A DNA molecule as claimed in claim 4, said DNA molecule being described by Figure I-1, I-2, IV-1, IV-2, IV-3, VI-1, VI-2 or VI-3.

14. A host cell transfected with a recombinant DNA molecule as claimed in any of claims 4 to 13.

15. A host cell co-transfected with a first recombinant DNA molecule and a second recombinant DNA molecule, said first recombinant DNA molecule being a recombinant DNA molecule as claimed in any one of claims 4 to 13 and said second recombinant DNA molecule encoding a peptide including the amino acid sequence of the HBV S peptide.

16. A host cell as claimed in claim 14 or 15 which is of eukaryotic origin.

17. A host cell as claimed in claim 16 which is a yeast cell.

18. A host cell as claimed in claim 16 which is of mammalian origin.

19. A host cell as claimed in claim 18 which is selected from a VERO cell, a 3T3 cell, a C127 cell, an L cell, a CHO cell which is positive for dehydrofolate reductase and a CHO cell which is negative for dehydrofolate reducatase.

20. A peptide encoded by a recombinant DNA molecule as claimed in any of claims 4 to 13.

21. A peptide as claimed in claim 20 for use in a method of vaccination against HBV.

22. A process for the manufacture of a medicament for vaccination against HBV **characterised in** the use, as an essential constituent of said medicament, of a peptide as claimed in claim 20.

## Patentansprüche (Patentansprüche für folgende(n) Vertragsstaat(en): AT, BE, CH, LI, DE, FR, GH, IT, LU, NL und SE)

1. Rekombinantes DNA-Molekül, umfassend eine Expressionskontrollsequenz, welche mit einer ersten und einer zweiten DNA-Sequenz funktionell verbunden ist, die im Leseraster verknüpft sind, wobei die DNA-Sequenzen jeweils einen getrennten Bereich eines einzelnen Peptids codieren, das von dem rekombinanten DNA-Molekül exprimiert wird, und wobei die erste DNA-Sequenz der Expressionskontrollsequenz näher ist als die zweite DNA-Sequenz, **dadurch gekennzeichnet, daß**:
(a) die erste DNA-Sequenz die Nucleotidsequenz eines Teils des HBV-prä-S₁-codierenden Bereichs umfaßt, worin ein 5'-terminaler Bereich des prä-S₁-codierenden Bereichs, enthaltend das ATG-Startcodon des prä-S₁-Bereichs, durch einen 5'-terminalen, codierenden Bereich des HBV-S-Bereichs, enthaltend das ATG-Startcodon des S-Bereichs, ersetzt wurde, und wobei die erste DNA-Sequenz ein HBV-prä-S₁-Epitop codiert; und
(b) die zweite DNA-Sequenz die Nucleotidsequenz des HBV-S-codierenden Bereichs umfaßt, worin das ATG-Startcodon fehlt, und ein Peptid codiert, das bei der Sekretion die Bildung von Partikeln steuert, die eine Immunantwort gegen das prä-S₁-Epitop auslösen.

2. DNA-Molekül, wie in Anspruch 1 beansprucht, worin die Nucleotidsequenz des 5'-terminalen, codierenden Bereichs des HBV-S-Bereichs ATGGAGAAC ist.

3. DNA-Molekül, wie in Anspruch 2 beansprucht, worin die Nucleotidsequenz des 5'-terminalen, codierenden Bereichs des HBV-S-Bereichs AACATGGAGAAC ist.

4. DNA-Molekül, wie in irgendeinem der Ansprüche 1 bis 3 beansprucht, wobei die erste DNA-Sequenz die Nucleotidsequenz eines Teils des HBV-prä-S₁-codierenden Bereichs umfaßt, worin der 3'-Terminus des prä-S₁-codierenden Bereichs deletiert wurde.

5. DNA-Molekül, wie in irgendeinem der Ansprüche 1 bis 4 beansprucht, das ein HBV-prä-S₁-Epitop des ayw-Serotyps codiert.

6. DNA-Molekül, wie in irgendeinem der Ansprüche 1 bis 5 beansprucht, enthaltend mindestens eine Nucleotidsequenz aus Tabelle I.

7. DNA-Molekül, wie in Anspruch 6 beansprucht, worin die erste DNA-Sequenz die Sequenz von Oligo Nr. 55 aus Tabelle I umfaßt.

8. DNA-Molekül, wie in Anspruch 6 beansprucht, woein die erste DNA-Sequenz die Sequenz von Oligo Nr. 23 aus Tabelle I umfaßt.

9. DNA-Molekül, wie in Anspruch 7 oder Anspruch 8 beansprucht, worin die zweite DNA-Sequenz die S-codierende Sequenz des XbaI-BglII-Fragments des in Figur IX gezeigten HBV-S-Gens umfaßt.

10. DNA-Molekül, wie in Anspruch 1 beansprucht, wobei das DNA-Molekül durch Figur I-1, I-2, IV-1, IV-2, IV-3, VI-1, VI-2 oder VI-3 beschrieben wird.

11. Wirtszelle, transfiziert mit einem rekombinanten DNA-Molekül, wie in irgendeinem der Ansprüche 1 bis 10 beansprucht.

12. Wirtszelle, cotransfiziert mit einem ersten rekombinanten DNA-Molekül und einem zweiten rekombinanten DNA-Molekül, wobei das erste rekombinante DNA-Molekül ein rekombinantes DNA-Molekül ist, wie in irgendeinem der Ansprüche 1 bis 10 beansprucht, und wobei das zweite rekombinante DNA-Molekül ein Peptid codiert, das die Aminosäuresequenz des HBV-S-Peptids einschließt.

13. Wirtszelle, wie in Anspruch 11 oder 12 beansprucht, die eukaryontischen Ursprungs ist.

14. Wirtszelle, wie in Anspruch 13 beansprucht, die eine Hefezelle ist.

15. Wirtszelle, wie in Anspruch 13 beansprucht, die von einem Säuger stammt.

16. Wirtszelle, wie in Anspruch 15 beansprucht, die aus einer VERO-Zelle, einer 3T3-Zelle, einer C127-Zelle, einer L-Zelle, einer CHO-Zelle, die für Dehydrofolatreduktase positiv ist, und einer CHO-Zelle, die für Dehydrofolatreduktase negativ ist, gewählt ist.

17. Peptid, codiert durch ein rekombinantes DNA-Molekül, wie in irgendeinem der Ansprüche 1 bis 10 beansprucht.

18. Peptid, wie in Anspruch 17 beansprucht, zur Verwendung in einem Verfahren zur Impfung gegen HBV.

19. Verfahren zur Herstellung einer transfizierten Wirtszelle, wobei das Verfahren umfaßt:
Bereitstellung einer Wirtszelle, die für die Aufnahme von DNA kompetent gemacht wurde;
Aussetzen der Wirtszelle einer ersten DNA-Zubereitung, umfassend ein rekombinantes DNA-Molekül nach irgendeinem der Ansprüche 1 bis 10;
Ermöglichen der DNA-Aufnahme aus der ersten DNA-Zubereitung durch die Wirtszelle unter geeigneten Bedingungen; und
Selektion auf eine Wirtszelle, die das rekombinante DNA-Molekül aufgenommen hat.

20. Verfahren, wie in Anspruch 19 beansprucht, weiterhin umfassend das Aussetzen der Wirtszelle einer zweiten DNA-Zubereitung, umfassend ein DNA-Molekül, das ein Peptid codiert, welches die Aminosäuresequenz des HBV-S-Peptids einschließt, und das Ermöglichen der DNA-Aufnahme aus der zweiten DNA-Zubereitung durch die Wirtszelle unter geeigneten Bedingungen.

21. Verfahren zur Herstellung eines Peptids, wobei das Verfahren die Züchtung einer Wirtszelle, transfiziert mit einem rekombinanten DNA-Molekül, wie in irgendeinem der Ansprüche 1 bis 10 beansprucht, unter Bedingungen, welche die Expression und Sekretion eines Proteins durch die Zelle zulassen, und die Gewinnung des als Ergebnis der Expression des rekombinanten DNA-Moleküls produzierten Peptids umfaßt.

## Patentansprüche (Patentansprüche für folgende(n) Vertragsstaat(en): ES und GR)

1. Verfahren zur Herstellung einer transfizierten Wirtszelle, wobei das Verfahren umfaßt:
Bereitstellung einer Wirtszelle, die für die Aufnahme von DNA kompetent gemacht wurde;
Aussetzen der Wirtszelle einer ersten DNA-Zubereitung, umfassend ein rekombinantes DNA-Molekül;
Ermöglichen der DNA-Aufnahme aus der ersten DNA-Zubereitung durch die Wirtszelle unter geeigneten Bedingungen; und
Selektion auf eine Wirtszelle, die das rekombinante DNA-Molekül aufgenommen hat,
wobei das rekombinante DNA-Molekül eine Expressionskontrollsequenz umfaßt, die mit einer ersten und einer zweiten DNA-Sequenz funktionell verbunden ist, die im Leseraster verknüpft sind, wobei die DNA-Sequenzen jeweils einen getrennten Bereich eines einzelnen Peptids codieren, das von dem rekombinanten DNA-Molekül exprimiert wird, und wobei die erste DNA-Sequenz der Expressionskontrollsequenz näher ist als die zweite DNA-Sequenz, **dadurch gekennzeichnet, daß**:
(a) die erste DNA-Sequenz die Nucleotidsequenz eines Teils des HBV-prä-S₁-codierenden Bereichs umfaßt, worin ein 5'-terminaler Bereich des prä-S₁-codierenden Bereichs, enthaltend das ATG-Startcodon des prä-S₁-Bereichs, durch einen 5'-terminalen, codierenden Bereich des HBV-S-Bereichs, enthaltend das ATG-Startcodon des S-Bereichs, ersetzt wurde, und wobei die erste DNA-Sequenz ein HBV-prä-S₁-Epitop codiert; und
(b) die zweite DNA-Sequenz die Nucleotidsequenz des HBV-S-codierenden Bereichs umfaßt, worin das ATG-Startcodon fehlt, und ein Peptid codiert, das bei der Sekretion die Bildung von Partikeln steuert, die eine Immunantwort gegen das prä-S₁-Epitop auslösen.

2. Verfahren, wie in Anspruch 1 beansprucht, weiterhin umfassend das Aussetzen der Wirtszelle einer zweiten DNA-Zubereitung, umfassend ein DNA-Molekül, das ein Peptid codiert, welches die Aminosäuresequenz des HBV-S-Peptids einschließt, und das Ermöglichen der DNA-Aufnahme aus der zweiten DNA-Zubereitung durch die Wirtszelle unter geeigneten Bedingungen.

3. Verfahren zur Herstellung eines Peptids, wobei das Verfahren die Züchtung einer Wirtszelle, transfiziert mit einem rekombinanten DNA-Molekül, unter Bedingungen, welche die Expression und Sekretion eines Proteins durch die Zelle zulassen, und die Gewinnung des als Ergebnis der Expression des rekombinanten DNA-Moleküls produzierten Peptids umfaßt, wobei das rekombinante DNA-Molekül eine Expressionskontrollsequenz umfaßt, die mit einer ersten und einer zweiten DNA-Sequenz funktionell verbunden ist, die im Leseraster verknüpft sind, wobei die DNA-Sequenzen jeweils einen getrennten Bereich eines einzelnen Peptids codieren, das von dem rekombinanten DNA-Molekül exprimiert wird, und wobei die erste DNA-Sequenz der Expressionskontrollsequenz näher ist als die zweite DNA-Sequenz, **dadurch gekennzeichnet, daß**:
(a) die erste DNA-Sequenz die Nucleotidsequenz eines Teils des HBV-prä-S₁-codierenden Bereichs umfaßt, worin ein 5'-terminaler Bereich des prä-S₁-codierenden Bereichs, enthaltend das ATG-Startcodon des prä-S₁-Bereichs, durch einen 5'-terminalen, codierenden Bereich des HBV-S-Bereichs, enthaltend das ATG-Startcodon des S-Bereichs, ersetzt wurde, und wobei die erste DNA-Sequenz ein HBV-prä-S₁-Epitop codiert; und
(b) die zweite DNA-Sequenz die Nucleotidsequenz des HBV-S-codierenden Bereichs umfaßt, worin das ATG-Startcodon fehlt, und ein Peptid codiert, das bei der Sekretion die Bildung von Partikeln steuert, die eine Immunantwort gegen das prä-S₁-Epitop auslösen.

4. Rekombinantes DNA-Molekül, umfassend eine Expressionskontrollsequenz, die mit einer ersten und einer zweiten DNA-Sequenz funktionell verbunden ist, die im Leseraster verknüpft sind, wobei die DNA-Sequenzen jeweils einen getrennten Bereich eines einzelnen Peptids codieren, das von dem rekombinanten DNA-Molekül exprimiert wird, und wobei die erste DNA-Sequenz der Expressionskontrollsequenz näher ist als die zweite DNA-Sequenz, **dadurch gekennzeichnet, daß**:
(a) die erste DNA-Sequenz die Nucleotidsequenz eines Teils des HBV-prä-S₁-codierenden Bereichs umfaßt, worin ein 5'-terminaler Bereich des prä-S₁-codierenden Bereichs, enthaltend das ATG-Startcodon des prä-S₁-Bereichs, durch einen 5'-terminalen, codierenden Bereich des HBV-S-Bereichs, enthaltend das ATG-Startcodon des S-Bereichs, ersetzt wurde, und wobei die erste DNA-Sequenz ein HBV-prä-S₁-Epitop codiert; und
(b) die zweite DNA-Sequenz die Nucleotidsequenz des HBV-S-codierenden Bereichs umfaßt, worin das ATG-Startcodon fehlt, und ein Peptid codiert, das bei der Sekretion die Bildung von Partikeln steuert, die eine Immunantwort gegen das prä-S₁-Epitop auslösen.

5. DNA-Molekül, wie in Anspruch 4 beansprucht, worin die Nucleotidsequenz des 5'-terminalen, codierenden Bereichs des HBV-S-Bereichs ATGGAGAAC ist.

6. DNA-Molekül, wie in Anspruch 5 beansprucht, worin die Nucleotidsequenz des 5'-terminalen, codierenden Bereichs des HBV-S-Bereichs AACATGGAGAAC ist.

7. DNA-Molekül, wie in irgendeinem der Ansprüche 4 bis 6 beansprucht, wobei die erste DNA-Sequenz die Nucleotidsequenz eines Teils des HBV-prä-S₁-codierenden Bereichs umfaßt, worin der 3'-Terminus des prä-S₁-codierenden Bereichs deletiert wurde.

8. DNA-Molekül, wie in irgendeinem der Ansprüche 4 bis 7 beansprucht, das ein HBV-prä-S₁-Epitop des ayw-Serotyps codiert.

9. DNA-Molekül, wie in irgendeinem der Ansprüche 4 bis 8 beansprucht, enthaltend mindestens eine Nucleotidsequenz aus Tabelle I.

10. DNA-Molekül, wie in Anspruch 9 beansprucht, worin die erste DNA-Sequenz die Sequenz von Oligo Nr. 55 aus Tabelle I umfaßt.

11. DNA-Molekül, wie in Anspruch 9 beansprucht, worin die erste DNA-Sequenz die Sequenz von Oligo Nr. 23 aus Tabelle I umfaßt.

12. DNA-Molekül, wie in Anspruch 10 oder Anspruch 11 beansprucht, worin die zweite DNA-Sequenz die S-codierende Sequenz des XbaI-BglII-Fragments des in Figur IX gezeigten HBV-S-Gens umfaßt.

13. DNA-Molekül, wie in Anspruch 4 beansprucht, wobei das DNA-Molekül durch Figur I-1, I-2, IV-1, IV-2, IV-3, VI-1, VI-2 oder VI-3 beschrieben wird.

14. Wirtszelle, transfiziert mit einem rekombinanten DNA-Molekül, wie in irgendeinem der Ansprüche 4 bis 13 beansprucht.

15. Wirtszelle, cotransfiziert mit einem ersten rekombinanten DNA-Molekül und einem zweiten rekombinanten DNA-Molekül, wobei das erste rekombinante DNA-Molekül ein rekombinantes DNA-Molekül ist, wie in irgendeinem der Ansprüche 4 bis 13 beansprucht, und wobei das zweite rekombinante DNA-Molekül ein Peptid codiert, welches die Aminosäuresequenz des HBV-S-Peptids einschließt.

16. Wirtszelle, wie in Anspruch 14 oder 15 beansprucht, die eukaryontischen Ursprungs ist.

17. Wirtszelle, wie in Anspruch 16 beansprucht, die eine Hefezelle ist.

18. Wirtszelle, wie in Anspruch 16 beansprucht, die von einem Säuger stammt.

19. Wirtszelle, wie in Anspruch 18 beansprucht, die aus einer VERO-Zelle, einer 3T3-Zelle, einer C127-Zelle, einer L-Zelle, einer CHO-Zelle, die für Dehydrofolatreduktase positiv ist, und einer CHO-Zelle, die für Dehydrofolatreduktase negativ ist, gewählt ist.

20. Peptid, codiert durch ein rekombinantes DNA-Molekül, wie in irgendeinem der Ansprüche 4 bis 13 beansprucht.

21. Peptid, wie in Anspruch 20 beansprucht, zur Verwendung in einem Verfahren zur Impfung gegen HBV.

22. Verfahren zur Herstellung eines Arzneimittels für die Impfung gegen HBV, das durch die Verwendung eines Peptids, wie in Anspruch 20 beansprucht, als einen wesentlichen Bestandteil des Arzneimittels **gekennzeichnet** ist.

## Revendications (Revendications pour l'(les) Etat(s) contractant(s) suivant(s): AT, BE, CH, LI, DE, FR, GB, IT, LU, NL, SE)

1. Une molécule d'ADN recombiné comprenant une séquence de régulation de l'expression liée de manière fonctionnelle à des première et seconde séquences d'ADN liées dans le même cadre de lecture, lesdites séquences d'ADN codant chacune une région discrète d'un seul peptide exprimé par ladite molécule d'ADN recombiné, et ladite première séquence d'ADN étant plus proche de ladite séquence de régulation de l'expression que la dite seconde séquence d'ADN, **caractérisée en ce que** :
(a) ladite première séquence d'ADN comprend la séquence nucléotidique d'une partie de la région codante pré-S₁ du VHB dans laquelle une région terminale 5' de ladite région codante pré-S₁ comprenant le codon initiateur ATG de la région pré-S₁ a été remplacé par une région codante 5'-terminale de la région S du VHB contenant le codon initiateur ATG de la région S, et ladite première séquence d'ADN code un épitope pré-S1 de VHB ; et
(b) ladite seconde séquence d'ADN comprend une séquence nucléotidique de la région codante S du VHB dans laquelle le codon initiateur ATG est absent et code un peptide qui par sécrétion dirigera la formation de particules qui provoqueront une réponse immunitaire contre l'épitope pré-S1.

2. Une molécule d'ADN telle que revendiquée dans la revendication 1, dans laquelle la séquence nucléotidique de ladite région codante 5'-terminale de la région S de VHB est ATGGAGAAC.

3. Une molécule d'ADN telle que revendiquée dans la revendication 2 dans laquelle la séquence nucléotidique de ladite région codante 5'-terminale de la région S de VHB est AACATGGAGAAC.

4. Une molécule d'ADN telle que revendiquée dans l'une quelconque des revendications 1 à 3, dans laquelle la première séquence d'ADN comprend la séquence nucléotidique d'une partie de la région codante pré-S₁ de VHB dans laquelle l'extrémité 3'-terminale de la région codante pré-S₁ a été délétée.

5. Une molécule d'ADN telle que revendiquée dans l'une quelconque des revendications 1 à 4 codant pour un épitope pré-S₁ de VHB du sérotype ayw.

6. Une molécule d'ADN telle que revendiquée dans l'une quelconque des revendications 1 à 5, contenant au moins une séquence nucléotidique du Tableau I.

7. Une molécule d'ADN telle que revendiquée dans la revendication 6, dans laquelle ladite première séquence d'ADN comprend la séquence d'oligo N° 55 du Tableau I.

8. Une molécule d'ADN telle que revendiquée dans la revendication 6, dans laquelle ladite première séquence d'ADN comprend la séquence d'oligo N° 23 du Tableau I.

9. Une molécule d'ADN telle que revendiquée dans la revendication 7 ou la revendication 8, dans laquelle ladite seconde séquence d'ADN comprend la séquence codante S du fragment XbaI-Bgl II du gène S du VHB tel que représenté dans la figure IX.

10. Une molécule d'ADN telle que revendiquée dans la revendication 4, ladite molécule d'ADN étant décrite dans la Figure I-1, I-2, IV-1, IV-2, IV-3, VI-1, VI-2 ou VI-3.

11. Une cellule hôte transfectée avec une molécule d'ADN recombiné telle que revendiquée dans l'une quelconque des revendications 1 à 10.

12. Une cellule hôte co-transfectée avec une première molécule d'ADN recombiné et une seconde molécule d'ADN recombiné, ladite première molécule d'ADN recombiné étant une molécule d'ADN recombiné telle que revendiquée dans l'une quelconque des revendications 1 à 10 et ladite seconde molécule d'ADN recombiné codant un peptide incluant la séquence d'acides aminés du peptide S de VHB.

13. Une cellule hôte telle que revendiquée dans la revendication 11 ou 12, qui est d'origine eucaryote.

14. Une cellule hôte telle que revendiquée dans la revendication 13, qui est une cellule de levure.

15. Une cellule hôte telle que revendiquée dans la revendication 13, qui est une cellule d'origine mammifère.

16. Une cellule hôte telle que revendiquée dans la revendication 15 qui est choisie parmi une cellule VERO, une cellule 3T3, une cellule C127, une cellule L, une cellule CHO qui est positive pour la déhydrofolate réductase et une cellule CHO qui est négative pour la déhydrofolate réductase.

17. Un peptide codé par une molécule d'ADN recombiné telle que revendiquée dans l'une quelconque des revendications 1 à 10.

18. Un peptide tel que revendiqué dans la revendication 17 pour utilisation dans une méthode de vaccination contre le VHB.

19. Un procédé pour produire une cellule hôte transfectée, ledit procédé comprenant le fait :
d'obtenir une cellule hôte qui a été rendue compétente pour l'absorption d'ADN,
d'exposer ladite cellule hôte à une première préparation d'ADN comprenant une molécule d'ADN recombiné de l'une quelconque des revendications 1 à 10,
de permettre à ladite cellule hôte, dans des conditions appropriées, d'absorber de l'ADN à partir de ladite première préparation d'ADN, et
de sélectionner une cellule hôte qui a absorbé ladite molécule d'ADN recombiné.

20. Un procédé tel que revendiqué dans la revendication 19, comprenant en outre le fait d'exposer ladite cellule hôte à une seconde préparation d'ADN comprenant une molécule d'ADN codant un peptide comprenant la séquence d'acides aminés du peptide S de VHB, et de permettre à ladite cellule hôte, dans des conditions appropriées, d'absorber l'ADN de ladite seconde préparation d'ADN.

21. Une méthode de production d'un peptide, ladite méthode comprenant le fait de cultiver une cellule hôte transfectée avec une molécule d'ADN recombiné telle que revendiquée dans l'une quelconque des revendications 1 à 10 dans des conditions permettant l'expression et la sécrétion de protéine par ladite cellule, et de recueillir le peptide produit à la suite de l'expression de ladite molécule d'ADN recombiné.

## Revendications (Revendications pour l'(les) Etat(s) contractant(s) suivant(s): ES et GR)

1. Un procédé pour produire une cellule hôte transfectée, ledit procédé comprenant le fait :
d'obtenir une cellule hôte qui a été rendue compétente pour l'absorption d'ADN,
d'exposer ladite cellule hôte à une première préparation d'ADN comprenant une molécule d'ADN recombiné,
de permettre à ladite cellule hôte, dans des conditions appropriées, d'absorber de l'ADN à partir de ladite première préparation d'ADN, et
de sélectionner une cellule hôte qui a absorbé ladite molécule d'ADN recombiné,
dans lequel ladite molécule d'ADN recombiné comprend une séquence de régulation de l'expression liée de manière fonctionnelle à des première et seconde séquences d'ADN liées dans le même cadre de lecture, lesdites séquences d'ADN codant chacune une région discrète d'un seul peptide exprimé par ladite molécule d'ADN recombiné, et ladite première séquence d'ADN étant plus proche de ladite séquence de régulation de l'expression que ladite seconde séquence d'ADN,
**caractérisé en ce que** :
(a) ladite première séquence d'ADN comprend la séquence nucléotidique d'une partie de la région codante pré-S₁ du VHB dans laquelle une région 5'-terminale de ladite région codante pré-S₁ contenant le codon initiateur ATG de la région pré-S₁, a été remplacé par une région codante 5'-terminale de la région S du VHB contenant le codon initiateur ATG de la région S, et ladite première séquence d'ADN code un épitope pré-S₁ de VHB ; et
(b) ladite première séquence d'ADN comprend une séquence nucléotidique de la région codante S du VHB dans laquelle le codon initiateur ATG est absent et code un peptide qui par sécrétion dirigera la formation de particules qui provoqueront une réponse immunitaire contre l'épitope pré-S₁.

2. Un procédé tel que revendiqué dans la revendication 1, comprenant en outre le fait d'exposer ladite cellule hôte à une seconde préparation d'ADN comprenant une molécule d'ADN codant un peptide comprenant la séquence d'acides aminés du peptide S de VHB, et de permettre à ladite cellule hôte, dans des conditions appropriées, d'absorber l'ADN de ladite seconde préparation d'ADN.

3. Une méthode de production d'un peptide, ladite méthode comprenant le fait de cultiver une cellule hôte transfectée avec une molécule d'ADN recombiné dans des conditions permettant l'expression et la sécrétion de protéine par ladite cellule, et de recueillir le peptide produit à la suite de l'expression de ladite molécule d'ADN recombiné, dans laquelle la dite molécule d'ADN recombiné comprend une séquence de régulation de l'expression liée de manière fonctionnelle à des première et seconde séquences d'ADN liées dans le même cadre de lecture, lesdites séquences d'ADN codant chacune une région discrète d'un seul peptide exprimé par ladite molécule d'ADN recombiné, et ladite première séquence d'ADN étant plus proche de la séquence de régulation de l'expression que ladite seconde séquence d'ADN, **caractérisée en ce que** :
(a) ladite première séquence d'ADN comprend la séquence nucléotidique d'une partie de la région codante pré-S₁ du VHB dans laquelle une région 5'-terminale de ladite région codante pré-S₁ contenant le codon initiateur ATG de la région pré-S₁ a été remplacée par une région codante 5' terminale de la région S de VHB contenant le codon initiateur ATG de la région S, et ladite première séquence d'ADN code un épitope pré-S₁ de VHB ; et
(b) ladite seconde séquence d'ADN comprend une séquence nucléotidique de la région codante S de VHB dans laquelle le codon initiateur ATG est absent et code un peptide qui par sécrétion conduira à la formation de particules qui provoqueront une réponse immunitaire contre l'épitope pré-S₁.

4. Une molécule d'ADN recombiné comprenant une séquence de régulation de l'expression liée de manière fonctionnelle à des première et seconde séquences d'ADN liées dans le même cadre de lecture, lesdites séquences d'ADN codant chacune une région discrète d'un seul peptide exprimé par ladite molécule d'ADN recombiné, et ladite première séquence d'ADN étant plus proche de ladite séquence de régulation de l'expression que la dite seconde séquence d'ADN, **caractérisée en ce que** :
(a) ladite première séquence d'ADN comprend la séquence nucléotidique d'une partie de la région codante pré-S₁ du VHB dans laquelle une région terminale 5' de ladite région codante pré-S₁ comprenant le codon initiateur ATG de la région pré-S₁ a été remplacé par une région codante 5'-terminale de la région S du VHB contenant le codon initiateur ATG de la région S, et ladite première séquence d'ADN code un épitope pré-S1 de VHB ; et
(b) ladite seconde séquence d'ADN comprend une séquence nucléotidique de la région codante S du VHB dans laquelle le codon initiateur ATG est absent et code un peptide qui par sécrétion dirigera la formation de particules qui provoqueront une réponse immunitaire contre l'épitope pré-S1.

5. Une molécule d'ADN telle que revendiquée dans la revendication 4, dans laquelle la séquence nucléotidique de ladite région codante 5'-terminale de la région S de VHB est ATGGAGAAC.

6. Une molécule d'ADN telle que revendiquée dans la revendication 5 dans laquelle la séquence nucléotidique de ladite région codante 5'-terminale de la région S de VHB est AACATGGAGAAC.

7. Une molécule d'ADN telle que revendiquée dans l'une quelconque des revendications 4 à 6, dans laquelle la première séquence d'ADN comprend la séquence nucléotidique d'une partie de la région codante pré-S₁ de VHB dans laquelle l'extrémité 3'-terminale de la région codante pré-S₁ a été délétée;

8. Une molécule d'ADN telle que revendiquée dans l'une quelconque des revendications 4 à 7 codant pour un épitope pré-S₁ de VHB du sérotype ayw.

9. Une molécule d'ADN telle que revendiquée dans l'une quelconque des revendications 4 à 8, contenant au moins une séquence nucléotidique du Tableau I.

10. Une molécule d'ADN telle que revendiquée dans la revendication 9, dans laquelle ladite première séquence d'ADN comprend la séquence d'oligo N° 55 du Tableau I.

11. Une molécule d'ADN telle que revendiquée dans la revendication 9, dans laquelle ladite première séquence d'ADN comprend la séquence d'oligo N° 23 du Tableau I.

12. Une molécule d'ADN telle que revendiquée dans la revendication 10 ou la revendication 11, dans laquelle ladite seconde séquence d'ADN comprend la séquence codante S du fragment XbaI-Bgl II du gène S du VHB tel que représenté dans la figure IX.

13. Une molécule d'ADN telle que revendiquée dans la revendication 4, ladite molécule d'ADN étant décrite dans la Figure I-1, I-2, IV-1, IV-2, IV-3, VI-1, VI-2 ou VI-3.

14. Une cellule hôte transfectée avec une molécule d'ADN recombiné telle que revendiquée dans l'une quelconque des revendications 4 à 13.

15. Une cellule hôte co-transfectée avec une première molécule d'ADN recombiné et une seconde molécule d'ADN recombiné, ladite première molécule d'ADN recombiné étant une molécule d'ADN recombiné telle que revendiquée dans l'une quelconque des revendications 4 à 13 et ladite seconde molécule d'ADN recombiné codant un peptide incluant la séquence d'acides aminés du peptide S de VHB.

16. Une cellule hôte telle que revendiquée dans la revendication 14 ou 15, qui est d'origine eucaryote.

17. Une cellule hôte telle que revendiquée dans la revendication 16, qui est une cellule de levure.

18. Une cellule hôte telle que revendiquée dans la revendication 16, qui est une cellule d'origine mammifère.

19. Une cellule hôte telle que revendiquée dans la revendication 16 qui est choisie parmi une cellule VERO, une cellule 3T3, une cellule C127, une cellule L, une cellule CHO qui est positive pour la déhydrofolate réductase et une cellule CHO qui est négative pour la déhydrofolate réductase.

20. Un peptide codé par une molécule d'ADN recombiné telle que revendiquée dans l'une quelconque des revendications 4 à 13.

21. Un peptide tel que revendiqué dans la revendication 20 pour utilisation dans une méthode de vaccination contre le VHB.

22. Un procédé pour la fabrication d'un médicament pour la vaccination contre le VHB, **caractérisé par** le fait d'utiliser, comme constituant essentiel dudit médicament, un peptide tel que revendiqué dans la revendication 20.
